(19) ![Europäisches Patentamt / European Patent Office / Office européen des brevets]

(11) **EP 4 613 880 A1**

(12) **EUROPEAN PATENT APPLICATION**
published in accordance with Art. 153(4) EPC

(43) Date of publication:
**10.09.2025 Bulletin 2025/37**

(21) Application number: **23886234.6**

(22) Date of filing: **31.10.2023**

(51) International Patent Classification (IPC):
**C12Q 1/6886** (2018.01)    **G16B 40/00** (2019.01)

(52) Cooperative Patent Classification (CPC):
**C12Q 1/6886; G16B 40/00**

(86) International application number:
**PCT/KR2023/017175**

(87) International publication number:
**WO 2024/096538 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.10.2022 KR 20220142050**

(71) Applicant: **GC Genome Corporation
Yongin-si, Gyeonggi-do 16924 (KR)**

(72) Inventors:
• **CHO, Eun-Hae
Yongin-si Gyeonggi-do 16924 (KR)**
• **KIM, Minjung
Yongin-si Gyeonggi-do 16924 (KR)**

(74) Representative: **Mewburn Ellis LLP
Aurora Building
Counterslip
Bristol BS1 6BX (GB)**

(54) **DNA METHYLATION MARKER FOR DIAGNOSING LIVER CANCER AND USES THEREOF**

(57)    The present invention relates to DNA methylation markers for diagnosing liver cancer and the use thereof, and more specifically, to a combination of DNA methylation markers capable of determining the presence or absence of liver cancer and the use thereof. The DNA methylation markers for diagnosing liver cancer according to the present invention are capable of diagnosing liver cancer with high accuracy using only DNA methylation information of a blood sample without using a liver cancer tissue sample, and thus may be useful for early diagnosis of liver cancer.

FIG. 1

EP 4 613 880 A1

# EP 4 613 880 A1

**Description**

**Technical Field**

The present invention relates to DNA methylation markers for diagnosing liver cancer and the use thereof, and more specifically, to a combination of DNA methylation markers capable of determining the presence or absence of liver cancer and the use thereof.

**Background Art**

Liver cancer is one of the most prevalent cancers worldwide. In Korea, the mortality rate of liver cancer is very high at 23 per 100,000 people, and about 10% of the total death rate in Korea is related to hepatitis, cirrhosis, and liver cancer. Liver cancer has no symptoms in the early stages, making early diagnosis difficult. Usually, liver cancer is mostly diagnosed in already advanced stages that cannot be properly treated, and thus treatment thereof is very limited and the prognosis thereof is also extremely poor. Since the prognosis of liver cancer varies greatly depending on the stage of the cancer at the time of diagnosis, early detection of liver cancer patients is very important in increasing the survival rate of liver cancer patients.

In order to accurately diagnose cancer, it is important not only to identify a mutated gene, but also to understand the mechanism by which the mutation occurs. Previously, studies were conducted focusing on mutations in the coding sequence of the gene, that is, micro-changes such as point mutations, deletions, and insertions, or on macroscopic chromosomal abnormalities. However, in recent years, extragenic changes have been reported to be as important as these mutations, and a representative example thereof is the methylation of promoter CpG islands.

In the genomic DNA of mammal cells, a fifth base, 5-methylcytosine (5-mC), in which a methyl group is attached to the fifth carbon of a cytosine ring is present in addition to A, C, G and T. 5-mC is always attached only to the C of a CG dinucleotide (5'-mCG-3'), and this CG is often denoted as CpG. The C of CpG is mostly methylated by attachment of a methyl group. This methylation of CpG inhibits the expression of repetitive sequences in the genome, such as Alu or transposons, and CpG is the site where extragenic changes most frequently occur in mammalian cells. 5-mC of this CpG is naturally converted into T through deamination. Accordingly, CpG appears in the mammalian genome with a frequency of only 1%, which is much lower than the normal frequency thereof ($\frac{1}{4}\times\frac{1}{4}$=6.25%).

There are regions called CpG islands in which CpGs are exceptionally dense. A CpG island is 0.2 to 3 kb in length, and is a highly concentrated region in which the distribution percentage of C and G bases is greater than 50% and the distribution percentage of CpG is 3.75% or more. About 45,000 CpG islands appear in the entire human genome and are concentrated in promoter regions, which regulate gene expression. Indeed, CpG islands appear in promoters of housekeeping genes, which account for about half of human genes (Cross S. et al., Curr. Opin. Gene Develop., 5:309, 1995). Accordingly, attempts have recently been actively made to investigate promoter methylation of tumor-related genes in blood, sputum, saliva, feces, urine, etc. and use the same to treat various types of cancer.

In current clinical practice, the diagnosis of cancer is made through history taking, physical examination, and clinical pathology tests, and once suspected, X-ray and endoscopic examinations are performed and tissue biopsy for confirmation is finally performed. However, the diagnosis of cancer by the current clinical test method is possible only when the number of cancer cells is 1 billion or more and the tumor diameter is 1 cm or more. In these cases, the cancer cells already have metastatic ability, and in half or more of the cases, the cancer has already metastasized. Meanwhile, tumor markers for detecting substances directly or indirectly produced by cancer are used in cancer screening, but they cause confusion due to limitations in accuracy, since up to about half thereof appear normal even in the presence of cancer, and they often appear positive even in the absence of cancer. Furthermore, anticancer agents that are mainly used for the treatment of cancer have a problem in that they exhibit an effect only when the volume of cancer is small.

Accordingly, methods of diagnosing cancer through DNA methylation measurement have recently been proposed. DNA methylation mainly occurs at cytosine of CpG islands in the promoter region of specific genes, and as a result, the binding of transcription factors is hindered, resulting in silencing of specific genes. This is the main mechanism by which the function of a gene is lost *in vivo* even in the absence of mutation in the protein-coding sequence of the gene, and is interpreted as the cause of the loss of function of many tumor suppressor genes in human cancer. Although there is controversy over whether methylation of promoter CpG islands directly causes carcinogenesis or is a secondary change leading to carcinogenesis, such abnormal methylation/demethylation in CpG islands has been reported in various cancer cells, including prostate cancer, colon cancer, uterine cancer, and breast cancer. Therefore, this abnormal methylation can be used in various ways, such as early diagnosis of cancer, prediction of carcinogenic risk, prediction of cancer prognosis, follow-up investigation after treatment, and prediction of response to anticancer therapy. Recently, there have been active attempts to use this abnormal methylation for cancer diagnosis and screening by investigating the abnormal methylation using methods such as methylation-specific PCR (hereinafter referred to as MSP), automated base analysis, or bisulfite pyrosequencing. However, most of these methods are limited to detecting and analyzing methylation of a small number of specific genes or promoter regions (e.g., Korean Patent No. 1557183, Korean Patent No. 1191947), and there are limitations in the efficiency and accuracy of diagnosis.

Accordingly, the present inventors have made extensive efforts to solve the above-described problems and develop DNA methylation markers for liver cancer diagnosis with high sensitivity and accuracy, and as a result, have simultaneously extracted features from TCGA methylation data of liver cancer tissue samples and cfDNA methylation data of liver cancer patients, and selected liver cancer-specific DNA methylation markers using a machine learning model that learned the features, and have found that, when the DNA methylation markers are analyzed, it is possible to diagnose liver cancer early with high accuracy, thereby completing the present invention.

**Summary of the Invention**

An object of the present invention is to provide a combination of DNA methylation markers for diagnosing liver cancer.

Another object of the present invention is to provide a method for providing information for diagnosing liver cancer using the combination of DNA methylation markers.

Still another object of the present invention is to provide a method for diagnosing liver cancer using the combination of DNA methylation markers.

Yet another object of the present invention is to provide a probe composition and a primer composition, which are capable of detecting the combination of DNA methylation markers, and a kit for diagnosing liver cancer including the composition.

To achieve the above objects, the present invention provides a combination of DNA methylation markers for diagnosing liver cancer, including DNA methylation markers shown in Table 1.

The present invention also provides a method for providing information for diagnosing liver cancer, including steps of: (a) isolating DNA from a biological sample; (b) detecting the methylation level of the combination of DNA methylation markers; and (c) determining that liver cancer is present if the detected DNA methylation marker level exceeds a cut-off value.

The present invention also provides a method for diagnosing liver cancer, including steps of: (a) isolating DNA from a biological sample; (b) detecting the methylation level of the combination of DNA methylation markers; and (c) determining that liver cancer is present if the detected DNA methylation marker level exceeds a cut-off value.

The present invention also provides a composition for diagnosing liver cancer, including a combination of primers capable of amplifying each DNA methylation marker of the combination of DNA methylation markers.

The present invention also provides a composition for diagnosing liver cancer, including a combination of probes, each capable of specifically hybridizing to either a polynucleotide including at least 10 contiguous nucleotides, which contains a methylated base of a DNA methylation marker of the combination of DNA methylation markers, or a polynucleotide complementary thereto.

The present invention also provides a kit for diagnosing liver cancer, including the composition.

**Brief Description of Drawings**

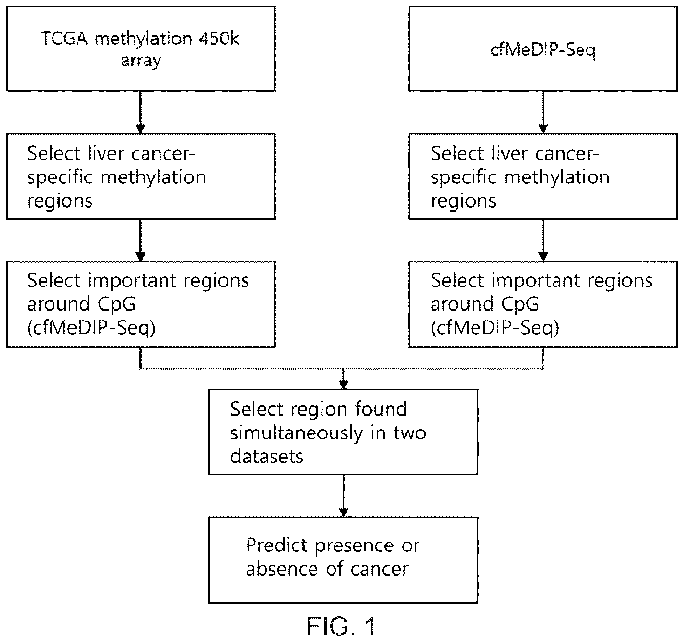

FIG. 1 is a flowchart showing the process of selecting DNA methylation markers for diagnosing liver cancers according to the present invention.

FIG. 2 is an ROC curve showing the results of evaluating the performance of a machine learning model trained using 354 liver cancer-specific DNA methylation markers selected according to one example of the present invention.

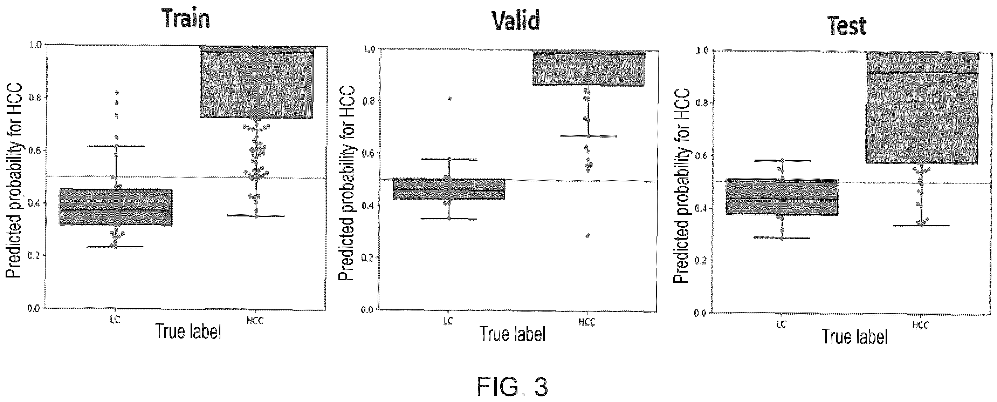

FIG. 3 is graphs showing the distribution of probability values calculated in the training, validation, and test groups to evaluate the performance of a machine learning model trained using 354 liver cancer-specific DNA methylation markers selected according to one example of the present invention.

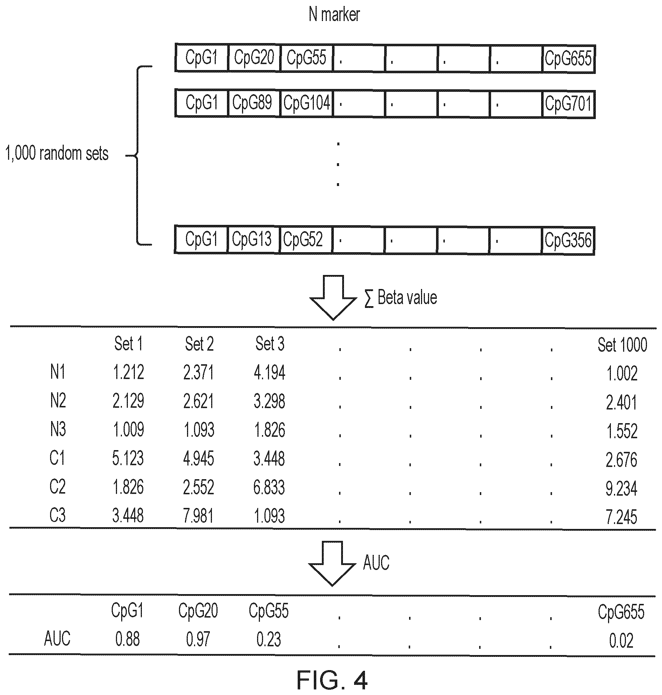

FIG. 4 is a flowchart showing the process of selecting a minimal combination of DNA methylation markers for diagnosing liver cancer according to the present invention.

FIG. 5 is a graph showing the difference in AUC values between 20 liver cancer-specific DNA methylation markers selected according to one example of the present invention and other marker set candidates.

FIG. 6 is an ROC_AUC graph showing the results of determining the presence or absence of liver cancer 20 liver cancer-specific DNA methylation markers selected according to one example of the present invention.

FIG. 7 shows the results of measuring the difference in methylation level between liver cancer and normal tissue for each of 20 liver cancer-specific DNA methylation markers selected according to one example of the present invention.

FIG. 8 is an ROC curve showing the results of evaluating the performance of a constructed machine learning model in clinical samples using 354 liver cancer-specific DNA methylation markers selected according to one example of the present invention.

FIG. 9 is graphs showing the distribution of probability values calculated in the training, validation, and test groups to evaluate the performance of a constructed machine learning model in clinical samples using 354 liver cancer-specific DNA methylation markers selected according to one example of the present invention.

FIG. 10 is an ROC curve showing the results of evaluating the performance of a constructed machine learning model in clinical samples using 14 liver cancer-specific DNA methylation markers selected according to one example of the present invention.

FIG. 11 is graphs showing the distribution of probability values calculated in the training, validation, and test groups to evaluate the performance of a constructed machine learning model in clinical samples using 14 liver cancer-specific DNA methylation markers selected according to one example of the present invention.

**Detailed Description and Preferred Embodiments of the Invention**

Unless otherwise defined, all technical and scientific terms used in the present specification have the same meanings as commonly understood by those skilled in the art to which the present disclosure pertains. In general, the nomenclature used in the present specification is well known and commonly used in the art.

In the present invention, it was sought to develop a model capable of diagnosing liver cancer using methylation information of cell-free nucleic acid in blood and confirm the accuracy thereof.

In the present invention, by combining methylation data of liver cancer tissue samples described in the TCGA database and methylation data of cell-free nucleic acids extracted from blood samples of liver cancer patients, a machine learning model was constructed and DNA methylation markers capable of determining the presence or absence of liver cancer were selected.

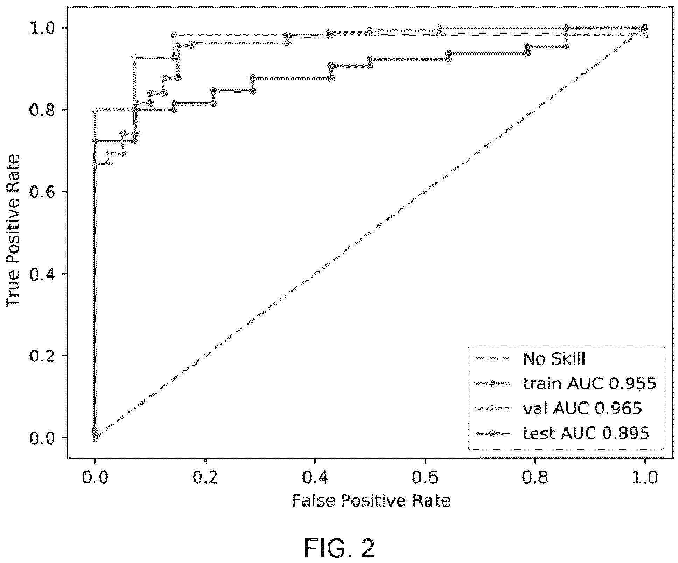

That is, in one example of the present invention, liver cancer-specific methylation regions were selected based on the methylation data of liver cancer tissue samples and normal samples described in the TCGA database, and methylated DNAs extracted from the blood of liver cancer patients and normal people were sequenced, and then compared to select liver cancer tissue-specific methylation regions. Then, overlapping regions between the selected regions were selected as the final input data, and it was confirmed that, when the presence or absence of liver cancer was determined by training a machine learning model using the final input data, it was possible to determine the presence or absence of liver cancer with high accuracy (FIG. 2).

Therefore, in one aspect,

the present invention relates to a combination of DNA methylation markers for diagnosing liver cancer, including DNA methylation markers shown in Table 1 below.

[Table 1]

| Chromosome | Start position | End position |
|---|---|---|
| chr1 | 119532189 | 119532190 |
| chr1 | 119532195 | 119532196 |
| chr1 | 146551744 | 146551745 |
| chr2 | 45160445 | 45160446 |
| chr4 | 41882163 | 41882164 |
| chr6 | 1624978 | 1624979 |
| chr6 | 26240579 | 26240580 |
| chr6 | 26252265 | 26252266 |
| chr7 | 27252541 | 27252542 |
| chr8 | 11540407 | 11540408 |
| chr14 | 54423433 | 54423434 |
| chr14 | 100632950 | 100632951 |
| chr17 | 80291775 | 80291776 |
| chr19 | 41317067 | 41317068 |

In the present invention, the combination of DNA methylation markers for diagnosing liver cancer may further include DNA markers shown in Table 2 below, without being limited thereto.

[Table 2]

| Chromosome | Start position | End position |
| --- | --- | --- |
| chr1 | 59042275 | 59042276 |
| chr2 | 208989248 | 208989249 |
| chr7 | 27225523 | 27225524 |
| chr10 | 77168431 | 77168432 |
| chr15 | 58357204 | 58357205 |
| chr18 | 32847566 | 32847567 |

In the present invention, the combination of DNA methylation markers for diagnosing liver cancer may further include two or more DNA methylation markers selected from the group consisting of DNA markers shown in Table 3 below, without being limited thereto.

[Table 3]

| Chromosome | Start position | End position | Chromosome | Start position | End position |
| --- | --- | --- | --- | --- | --- |
| chr1 | 46632446 | 46632945 | chr2 | 119067503 | 119068002 |
| chr1 | 46632621 | 46633120 | chr2 | 157177686 | 157178185 |
| chr1 | 47697715 | 47698214 | chr2 | 157178481 | 157178980 |
| chr1 | 47908984 | 47909483 | chr2 | 157178639 | 157179138 |
| chr1 | 47909931 | 47910430 | chr2 | 160761163 | 160761662 |
| chr1 | 47910206 | 47910705 | chr2 | 176987215 | 176987714 |
| chr1 | 47910593 | 47911092 | chr2 | 177003485 | 177003984 |
| chr1 | 48058627 | 48059126 | chr2 | 177003497 | 177003996 |
| chr1 | 48058711 | 48059210 | chr2 | 200331667 | 200332166 |
| chr1 | 67772878 | 67773377 | chr2 | 200331727 | 200332226 |
| chr1 | 87617460 | 87617959 | chr2 | 200331775 | 200332274 |
| chr1 | 91192216 | 91192715 | chr2 | 200333751 | 200334250 |
| chr1 | 110610649 | 110611148 | chr2 | 200334851 | 200335350 |
| chr1 | 119522605 | 119523104 | chr2 | 238583254 | 238583753 |
| chr1 | 119526861 | 119527360 | chr20 | 1784026 | 1784525 |
| chr1 | 119526906 | 119527405 | chr20 | 30778049 | 30778548 |
| chr1 | 119527634 | 119528133 | chr20 | 37433979 | 37434478 |
| chr1 | 119529680 | 119530179 | chr20 | 50720658 | 50721157 |
| chr1 | 119532523 | 119533022 | chr20 | 50721063 | 50721562 |
| chr1 | 119532675 | 119533174 | chr3 | 38080675 | 38081174 |
| chr1 | 119542807 | 119543306 | chr3 | 101497626 | 101498125 |
| chr1 | 119542966 | 119543465 | chr3 | 101497730 | 101498229 |
| chr1 | 119543086 | 119543585 | chr3 | 101497732 | 101498231 |
| chr1 | 119548277 | 119548776 | chr3 | 138658771 | 138659270 |
| chr1 | 119548575 | 119549074 | chr3 | 138662065 | 138662564 |
| chr1 | 119548602 | 119549101 | chr3 | 138662980 | 138663479 |

(continued)

| Chromosome | Start position | End position | Chromosome | Start position | End position |
|---|---|---|---|---|---|
| chr1 | 119548895 | 119549394 | chr3 | 142837745 | 142838244 |
| chr1 | 119549013 | 119549512 | chr3 | 147098318 | 147098817 |
| chr1 | 151812171 | 151812670 | chr3 | 147098335 | 147098834 |
| chr1 | 151812185 | 151812684 | chr3 | 147105760 | 147106259 |
| chr1 | 151812274 | 151812773 | chr3 | 147136654 | 147137153 |
| chr1 | 151812460 | 151812959 | chr3 | 169529777 | 169530276 |
| chr1 | 154474923 | 154475422 | chr3 | 169529787 | 169530286 |
| chr1 | 156130576 | 156131075 | chr3 | 179168510 | 179169009 |
| chr1 | 156389874 | 156390373 | chr3 | 179168548 | 179169047 |
| chr1 | 160951657 | 160952156 | chr3 | 183145282 | 183145781 |
| chr1 | 161275311 | 161275810 | chr3 | 186490406 | 186490905 |
| chr1 | 169396462 | 169396961 | chr4 | 785994 | 786493 |
| chr1 | 170629820 | 170630319 | chr4 | 41868836 | 41869335 |
| chr1 | 170630308 | 170630807 | chr4 | 41880497 | 41880996 |
| chr1 | 171810218 | 171810717 | chr4 | 41882330 | 41882829 |
| chr1 | 171810722 | 171811221 | chr4 | 76555297 | 76555796 |
| chr1 | 171811049 | 171811548 | chr4 | 76555384 | 76555883 |
| chr1 | 197882219 | 197882718 | chr4 | 76555522 | 76556021 |
| chr1 | 203598323 | 203598822 | chr4 | 76555527 | 76556026 |
| chr1 | 213123425 | 213123924 | chr4 | 76555532 | 76556031 |
| chr1 | 213123636 | 213124135 | chr5 | 7849953 | 7850452 |
| chr1 | 213123715 | 213124214 | chr5 | 7850188 | 7850687 |
| chr1 | 221064450 | 221064949 | chr5 | 32713473 | 32713972 |
| chr1 | 228645797 | 228646296 | chr5 | 40680887 | 40681386 |
| chr1 | 247171153 | 247171652 | chr5 | 40681643 | 40682142 |
| chr10 | 8094284 | 8094783 | chr5 | 42950942 | 42951441 |
| chr10 | 16562220 | 16562719 | chr5 | 42951863 | 42952362 |
| chr10 | 17271679 | 17272178 | chr5 | 42992524 | 42993023 |
| chr10 | 17271694 | 17272193 | chr5 | 43017435 | 43017934 |
| chr10 | 17271744 | 17272243 | chr5 | 43018193 | 43018692 |
| chr10 | 17271867 | 17272366 | chr5 | 54516555 | 54517054 |
| chr10 | 22541774 | 22542273 | chr5 | 94955506 | 94956005 |
| chr10 | 22625215 | 22625714 | chr5 | 112073100 | 112073599 |
| chr10 | 22765590 | 22766089 | chr5 | 112073123 | 112073622 |
| chr10 | 43697758 | 43698257 | chr5 | 112073148 | 112073647 |
| chr10 | 93647050 | 93647549 | chr5 | 112073156 | 112073655 |
| chr10 | 94834332 | 94834831 | chr5 | 112073176 | 112073675 |
| chr10 | 94834513 | 94835012 | chr5 | 112073188 | 112073687 |
| chr10 | 102894793 | 102895292 | chr5 | 139047755 | 139048254 |

(continued)

| Chromosome | Start position | End position | Chromosome | Start position | End position |
|---|---|---|---|---|---|
| chr10 | 104000581 | 104001080 | chr5 | 139047856 | 139048355 |
| chr11 | 13689872 | 13690371 | chr5 | 169064201 | 169064700 |
| chr11 | 13689910 | 13690409 | chr5 | 170736027 | 170736526 |
| chr11 | 13690452 | 13690951 | chr6 | 3228983 | 3229482 |
| chr11 | 31826324 | 31826823 | chr6 | 5026074 | 5026573 |
| chr11 | 69517049 | 69517548 | chr6 | 5026185 | 5026684 |
| chr11 | 69517221 | 69517720 | chr6 | 10425398 | 10425897 |
| chr11 | 69517591 | 69518090 | chr6 | 10425849 | 10426348 |
| chr11 | 69517753 | 69518252 | chr6 | 26235004 | 26235503 |
| chr11 | 69517947 | 69518446 | chr6 | 26240670 | 26241169 |
| chr12 | 21810029 | 21810528 | chr6 | 26250494 | 26250993 |
| chr12 | 21810380 | 21810879 | chr6 | 26250669 | 26251168 |
| chr12 | 21810558 | 21811057 | chr6 | 26250686 | 26251185 |
| chr12 | 48206598 | 48207097 | chr6 | 26251649 | 26252148 |
| chr12 | 58021319 | 58021818 | chr6 | 26271466 | 26271965 |
| chr12 | 58021463 | 58021962 | chr6 | 26271468 | 26271967 |
| chr12 | 81102236 | 81102735 | chr6 | 26271566 | 26272065 |
| chr12 | 95941619 | 95942118 | chr6 | 26271577 | 26272076 |
| chr12 | 95941738 | 95942237 | chr6 | 26550760 | 26551259 |
| chr12 | 95942511 | 95943010 | chr6 | 26614399 | 26614898 |
| chr12 | 95942657 | 95943156 | chr6 | 27462967 | 27463466 |
| chr12 | 95942714 | 95943213 | chr6 | 27858387 | 27858886 |
| chr12 | 115102476 | 115102975 | chr6 | 28411037 | 28411536 |
| chr13 | 45149778 | 45150277 | chr6 | 42738717 | 42739216 |
| chr13 | 45150012 | 45150511 | chr6 | 42738799 | 42739298 |
| chr13 | 100627091 | 100627590 | chr6 | 100911437 | 100911936 |
| chr13 | 100641159 | 100641658 | chr6 | 100912656 | 100913155 |
| chr13 | 100641396 | 100641895 | chr6 | 100912690 | 100913189 |
| chr13 | 107186620 | 107187119 | chr6 | 100912696 | 100913195 |
| chr13 | 107186927 | 107187426 | chr6 | 100915517 | 100916016 |
| chr14 | 51027611 | 51028110 | chr6 | 108488085 | 108488584 |
| chr14 | 54422525 | 54423024 | chr6 | 108490645 | 108491144 |
| chr14 | 61108957 | 61109456 | chr6 | 108495615 | 108496114 |
| chr15 | 33009281 | 33009780 | chr6 | 108495735 | 108496234 |
| chr15 | 41805280 | 41805779 | chr6 | 133561851 | 133562350 |
| chr15 | 55880644 | 55881143 | chr6 | 133562216 | 133562715 |
| chr15 | 65186056 | 65186555 | chr6 | 133562220 | 133562719 |
| chr15 | 68260324 | 68260823 | chr6 | 133562225 | 133562724 |
| chr15 | 69087559 | 69088058 | chr6 | 133562229 | 133562728 |

(continued)

| Chromosome | Start position | End position | Chromosome | Start position | End position |
|---|---|---|---|---|---|
| chr15 | 99193679 | 99194178 | chr6 | 133562235 | 133562734 |
| chr15 | 99193743 | 99194242 | chr6 | 133562242 | 133562741 |
| chr15 | 101459033 | 101459532 | chr6 | 133562244 | 133562743 |
| chr16 | 21170817 | 21171316 | chr7 | 27204478 | 27204977 |
| chr17 | 4981360 | 4981859 | chr7 | 27204731 | 27205230 |
| chr17 | 4981573 | 4982072 | chr7 | 27204967 | 27205466 |
| chr17 | 29297898 | 29298397 | chr7 | 27213734 | 27214233 |
| chr17 | 29297934 | 29298433 | chr7 | 27213793 | 27214292 |
| chr17 | 29298102 | 29298601 | chr7 | 27213806 | 27214305 |
| chr17 | 42030229 | 42030728 | chr7 | 27232587 | 27233086 |
| chr17 | 43338973 | 43339472 | chr7 | 27232823 | 27233322 |
| chr17 | 43339078 | 43339577 | chr7 | 27232891 | 27233390 |
| chr17 | 43339247 | 43339746 | chr7 | 27245292 | 27245791 |
| chr17 | 43339262 | 43339761 | chr7 | 27284539 | 27285038 |
| chr17 | 46655579 | 46656078 | chr7 | 27291096 | 27291595 |
| chr17 | 48636396 | 48636895 | chr7 | 28996389 | 28996888 |
| chr17 | 59529066 | 59529565 | chr7 | 28996652 | 28997151 |
| chr17 | 59529236 | 59529735 | chr7 | 28996923 | 28997422 |
| chr17 | 59534597 | 59535096 | chr7 | 28997235 | 28997734 |
| chr17 | 59534748 | 59535247 | chr7 | 28997616 | 28998115 |
| chr17 | 62777398 | 62777897 | chr7 | 28997828 | 28998327 |
| chr17 | 79480858 | 79481357 | chr7 | 76828635 | 76829134 |
| chr18 | 32847001 | 32847500 | chr7 | 96636366 | 96636865 |
| chr18 | 55019849 | 55020348 | chr7 | 96636496 | 96636995 |
| chr19 | 12305604 | 12306103 | chr7 | 96651031 | 96651530 |
| chr19 | 12305619 | 12306118 | chr7 | 96651865 | 96652364 |
| chr19 | 12305886 | 12306385 | chr7 | 96651873 | 96652372 |
| chr19 | 12305948 | 12306447 | chr7 | 117119174 | 117119673 |
| chr19 | 13209731 | 13210230 | chr7 | 117119351 | 117119850 |
| chr19 | 36736022 | 36736521 | chr7 | 117119361 | 117119860 |
| chr19 | 38182805 | 38183304 | chr7 | 117119387 | 117119886 |
| chr19 | 38754889 | 38755388 | chr7 | 117119688 | 117120187 |
| chr19 | 42901057 | 42901556 | chr7 | 134143656 | 134144155 |
| chr19 | 50553817 | 50554316 | chr7 | 134143669 | 134144168 |
| chr19 | 50554030 | 50554529 | chr7 | 134143786 | 134144285 |
| chr19 | 50554201 | 50554700 | chr7 | 143042491 | 143042990 |
| chr19 | 52207103 | 52207602 | chr7 | 143042548 | 143043047 |
| chr19 | 57018819 | 57019318 | chr7 | 151329758 | 151330257 |
| chr19 | 58220045 | 58220544 | chr8 | 41424092 | 41424591 |

(continued)

| Chromosome | Start position | End position | Chromosome | Start position | End position |
|---|---|---|---|---|---|
| chr19 | 58220120 | 58220619 | chr8 | 49292435 | 49292934 |
| chr2 | 20068452 | 20068951 | chr8 | 53851934 | 53852433 |
| chr2 | 25438860 | 25439359 | chr8 | 57069657 | 57070156 |
| chr2 | 45159843 | 45160342 | chr8 | 59058004 | 59058503 |
| chr2 | 45170072 | 45170571 | chr8 | 67873093 | 67873592 |
| chr2 | 45231217 | 45231716 | chr8 | 67873226 | 67873725 |
| chr2 | 45231532 | 45232031 | chr8 | 67873549 | 67874048 |
| chr2 | 45231641 | 45232140 | chr8 | 67873928 | 67874427 |
| chr2 | 45232167 | 45232666 | chr8 | 67874116 | 67874615 |
| chr2 | 63280819 | 63281318 | chr8 | 67874783 | 67875282 |
| chr2 | 63280889 | 63281388 | chr8 | 70981789 | 70982288 |
| chr2 | 63281067 | 63281566 | chr8 | 86350318 | 86350817 |
| chr2 | 63281133 | 63281632 | chr8 | 86350331 | 86350830 |
| chr2 | 63281594 | 63282093 | chr8 | 98289898 | 98290397 |
| chr2 | 63282452 | 63282951 | chr8 | 99951797 | 99952296 |
| chr2 | 63282763 | 63283262 | chr8 | 99959473 | 99959972 |
| chr2 | 63283717 | 63284216 | chr8 | 99959898 | 99960397 |
| chr2 | 63283816 | 63284315 | chr8 | 99961295 | 99961794 |
| chr2 | 63284518 | 63285017 | chr8 | 99961624 | 99962123 |
| chr2 | 63285799 | 63286298 | chr8 | 102504197 | 102504696 |
| chr2 | 74425262 | 74425761 | chr8 | 102504251 | 102504750 |
| chr2 | 74425330 | 74425829 | chr8 | 102504314 | 102504813 |
| chr2 | 74781846 | 74782345 | chr8 | 102505306 | 102505805 |
| chr2 | 105470311 | 105470810 | chr8 | 104512833 | 104513332 |
| chr2 | 119067387 | 119067886 | chr9 | 110228019 | 110228518 |

The term "DNA methylation" in the present invention refers to covalent bonding of a methyl group to the C5 position of a cytosine base in genomic DNA. The methylation level means, for example, the level of methylation present in a DNA base sequence in all genomic regions and some non-genomic regions, and in the present invention, it means the degree of methylation of the DNA methylation marker. In the DNA methylation marker, methylation may occur throughout the entire sequence or part of the sequence.

In the present invention, the liver cancer may be hepatocellular carcinoma originating from hepatocytes or metastatic liver cancer caused by metastasis of cancer from other tissues to the liver, but is preferably hepatocellular carcinoma. Hepatocellular carcinoma accounts for about 90% of malignant tumors that occur in the liver, and occurs frequently in Korea, Japan, Southeast Asia, China, etc. Hepatocellular carcinoma mostly occurs in the presence of liver cirrhosis, but is also caused by chronic hepatitis B or C in some cases.

In another aspect, the present invention relates to a method for providing information for diagnosing liver cancer, including steps of:

(a) isolating DNA from a biological sample;
(b) detecting the methylation level of the combination of DNA methylation markers; and
(c) determining that liver cancer is present if the detected DNA methylation marker level exceeds a cut-off value.

In another aspect, the present invention relates to a method for diagnosing liver cancer, including steps of:

(a) isolating DNA from a biological sample;

(b) detecting the methylation level of the combination of DNA methylation markers; and

(c) determining that liver cancer is present if the detected DNA methylation marker level exceeds a cut-off value.

In the present invention, the DNA may be, without limitation, a DNA extracted from a biological sample, but is preferably a fragment of cell-free nucleic acid or intracellular nucleic acid, without being limited thereto.

In the present invention, the biological sample means any material, biological fluid, tissue or cells obtained from or derived from a subject, and examples thereof include, but are not limited to, whole blood, leukocytes, peripheral blood mononuclear cells, leukocyte buffy coat, blood including plasma and serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, semen, hair, saliva, urine, oral cells, placental cells, cerebrospinal fluid, and mixtures thereof.

In the present invention, detecting the methylation level in step (b) may be performed by various known methods, and is preferably performed by bisulfite conversion or methylated DNA immunoprecipitation (MeDIP), without being limited thereto.

In the present invention, methods capable of detecting DNA methylation further include a restriction enzyme-based detection method, which is a method in which an unmethylated nucleic acid is cleaved using methylation restriction enzymes (MREs) or a specific sequence (recognition site) is cleaved regardless of methylation, and is analyzed using a hybridization method or PCR.

In the present invention, bisulfite conversion-based methods include whole-genome bisulfite sequencing (WGBS), reduced-representation bisulfite sequencing (RRBS), methylated CpG tandems amplification and sequencing (MCTA-seq), targeted bisulfite sequencing, methylation array, and methylation-specific PCR (MSP).

In the present invention, methods for enriching and analyzing methylated DNA include methylated DNA immunoprecipitation sequencing (MeDIP-seq), methyl-CpG binding domain protein capture sequencing (MBD-seq), and the like.

In the present invention, another method capable of analyzing methylated DNA is 5-hydroxymethylation profiling, examples of which include 5hmC-Seal (hMe-Seal), hmC-CATCH, hydroxymethylated DNA immunoprecipitation sequencing (hMeDIP-seq), and oxidative bisulfite conversion.

In the present invention, detecting the methylation level in step (b) may be performed using any one method selected from the group consisting of PCR, methylation-specific PCR, real time methylation-specific PCR, PCR using methylated DNA-specific binding protein, quantitative PCR, PCR using methylation-specific PNA, melting curve analysis, DNA chip assay, pyrosequencing, bisulfite sequencing, and methylation next-generation sequencing, without being limited thereto.

In the present invention, the next-generation sequencer may be used for any sequencing method known in the art. Sequencing of the nucleic acids isolated by the selection method is typically performed using next-generation sequencing (NGS). Next-generation sequencing includes any sequencing method that determines the nucleotide sequence of either individual nucleic acid molecules or clonally expanded proxies for individual nucleic acid molecules in a high throughput fashion (e.g., $10^5$ or more molecules are sequenced simultaneously). In one embodiment, the relative abundance of the nucleic acid species in the library may be estimated by counting the relative number of occurrences of their cognate sequences in the data generated by the sequencing experiment. Next generation sequencing methods are known in the art, and are described, e.g., in Metzker, M. (2010) Nature Biotechnology Reviews 11:31-46, which is incorporated herein by reference.

In one embodiment, the next-generation sequencing allows for the determination of the nucleotide sequence of an individual nucleic acid molecule (e.g., Helicos BioSciences' HeliScope Gene Sequencing system, and Pacific Biosciences' PacBio RS system). In other embodiments, the sequencing method determines the nucleotide sequence of clonally expanded proxies for individual nucleic acid molecules (e.g., the Solexa sequencer, Illumina Inc., San Diego, Calif.; 454 Life Sciences (Branford, Conn.), and Ion Torrent), e.g., massively parallel short-read sequencing (e.g., the Solexa sequencer, Illumina Inc., San Diego, Calif.), which generates more bases of sequence per sequencing unit than other sequencing methods that generate fewer but longer reads. Other methods or machines for next-generation sequencing include, but are not limited to, the sequencers provided by 454 Life Sciences (Branford, Conn.), Applied Biosystems (Foster City, Calif.; SOLiD sequencer), Helicos BioSciences Corporation (Cambridge, Mass.), and emulsion and microfluidic sequencing technology nanodroplets (e.g., GnuBio droplets).

Platforms for next-generation sequencing include, but are not limited to, Roche/454's Genome Sequencer (GS) FLX System, Illumina/Solexa's Genome Analyzer (GA), Life/APG's Support Oligonucleotide Ligation Detection (SOLiD) system, Polonator's G.007 system, Helicos BioSciences' HeliScope Gene Sequencing system, Oxford Nanopore Technologies' PromethION, GriION, MinION system, and Pacific Biosciences' PacBio RS system.

In the present invention, step (c) may be performed by a step of determining the presence or absence of liver cancer by comparing an output result value, obtained by inputting and analyzing information on the detected methylation level of the combination of DNA methylation markers into an artificial intelligence model trained to diagnose liver cancer, with a cut-off value, without being limited thereto.

In the present invention, the cut-off value in step (c) may be, without limitation, a value that may determine the presence or absence of liver cancer, and is preferably 0.5 to 1, more preferably 0.5 to 0.8, most preferably 0.5, without being limited thereto.

In the present invention, when an artificial intelligence model is used in step (c), the artificial intelligence model may be, without limitation, an artificial intelligence model that may determine the presence or absence of liver cancer, preferably a machine learning model. More preferably, it may be at least one machine learning model selected from the group consisting of K-nearest neighbors, linear regression, logistic regression, support vector machine (SVM), decision tree, random forest, and artificial neural network models, preferably an artificial neural network model, without being limited thereto.

In the present invention, the artificial neural network model may be selected from the group consisting of convolutional neural network (CNN), deep neural network (DNN), recurrent neural network (RNN), and autoencoder models, and may most preferably be a convolutional neural network model, without being limited thereto.

In the present invention, when the artificial intelligence model is a CNNmodel, the loss function for performing binary classification may be expressed by Equation 1 below.

$$\text{Equation 1: Binary classification}$$

$$\text{loss(model(x),y)} = -\frac{1}{n}\left[\sum_{i=1}^{n}\left(y_i\log\left(model(x_i)\right) + (1-y_i)\log\left(1-model(x_i)\right)\right)\right]$$

$$model(x_i) = artificial\ intelligence\ model\ output\ for\ i-th\ input$$
$$y = \text{actual label value}$$
$$n = \text{number of input data}$$

In the present invention, the binary classification means that the artificial intelligence model is trained to determine the presence or absence of cancer.

In the present invention, when the artificial intelligence model is a CNN model, training may include the following steps:

i) classifying information on the detected methylation level into training, validation, and test data, wherein the training data are used when the CNN model is trained, the validation data are used for validation of hyper-parameter tuning, and the test data are used for performance evaluation after generating the optimal model;
ii) constructing an optimal CNN model through hyper-parameter tuning and training processes; and
iii) comparing the performance of multiple models obtained through hyper-parameter tuning using the validation data and determining the model with the best validation data performance as the optimal model.

In the present invention, the hyper-parameter tuning process is a process of optimizing the values of various parameters (the number of convolution layers, the number of dense layers, the number of convolution filters, etc.) constituting the CNN model, and may be performed using Bayesian optimization and grid search techniques.

In the present invention, the internal parameters (weights) of the CNN model are optimized using predetermined hyper-parameters, and when validation loss starts to increase compared to training loss, the model is determined to be overfit, and training of the model is stopped prior to the overfitting.

In the present invention, the artificial intelligence model is trained to output a value close to 1 if there is cancer and to output a value close to 0 if there is no cancer. Therefore, performance (training, validation, and test accuracy) is measured based on a cut-off value of 0.5. In other words, if the output value is 0.5 or more, it is determined that there is cancer, and if it is less than 0.5, it is determined that there is no cancer.

Here, it will be apparent to those skilled in the art that the cut-off value of 0.5 may be arbitrarily changed. For example, in order to reduce false positives, the cut-off value may be set to be higher than 0.5 as a stricter criterion for determining cancer, and in order to reduce false negatives, the cut-off value may be set to be lower than 0.5 as a weaker criterion for determining cancer.

Most preferably, the cut-off value may be set by determining the probability of DPI by applying unseen data (data containing known answers that are different from those trained during learning) using the trained artificial intelligence model.

In the present invention, the value resulting from the data input to the artificial intelligence model in step (c) may be used, without limitation, a specific score or a real number, and preferably may be a deep probability index (DPI), without being limited thereto.

In the present invention, the deep probability index means a value expressed as a probability value by adjusting the output of the artificial intelligence model to a scale of 0 to 1 using the sigmoid function (Equation 2 below) for binary classification in the final layer of the artificial intelligence model.

$$\text{Equation 2: Sigmoid function}$$

$$\frac{1}{1+e^{-x}} \, , \; x = output \; layer$$

For binary classification, the model is trained using the sigmoid function so that the DPI becomes 1 when there is cancer. For example, when liver cancer samples and normal samples are input, the model is trained so that the DPI of the liver cancer samples is closer to 1.

In the present invention, step (c) may be performed by a step of comparing information on the detected methylation level of the combination of DNA methylation markers with the value of the normal samples and determining that liver cancer is present if the detected methylation level is greater than the cut-off value, without being limited thereto.

In the present invention, the cut-off value in step (c) may be, without limitation, a value that may be used to determine the presence or absence of liver cancer, and preferably, it may be 99% to 75%, more preferably 97% to 80%, most preferably 95%, of the methylation level of the normal samples, without being limited thereto.

In the present invention, in step (c), the information on the detected methylation level of the combination of DNA methylation markers may be one or more values selected from the group consisting of the sum, difference, product, mean, log of product, log of sum, median, quantile, minimum, maximum, variance, standard deviation, absolute deviation, coefficient of variation, reciprocal values thereof, and combinations thereof, of the beta values of the individual markers, without being limited thereto.

In the present invention, as is well known to those skilled in the art, when calculating the information on the methylation level as the beta value, the beta value of a hypermethylation marker is used as is, and the beta value of a hypomethylation marker is calculated by subtracting from a certain cut-off value such as 100 or 1, or by multiplying by -1.

In another aspect, the present invention relates to a composition for diagnosing liver cancer, including a combination of primers capable of amplifying each DNA methylation marker of the combination of DNA methylation markers.

In the present invention, the appropriate length of the primer may vary depending on the intended use, but may generally consist of 15 to 30 nucleotides. The primer sequence does not need to be completely complementary to the template, but should be sufficiently complementary to hybridize with the template. The primer is capable of hybridizing to a DNA sequence including a methylation marker and amplifying a DNA fragment including the methylation marker. The primer of the present invention may be used in a diagnostic kit or prediction method for determining the presence or absence of liver cancer by detecting the level of DNA methylation.

In the present invention, the primer capable of amplifying the DNA methylation marker may be, without limitation, a nucleotide sequence of the same chromosome that does not directly include the marker region, but specifically, it may include 1 to 1,000-bp 5' upstream and 1 to 1,000-bp 3' downstream of the marker region, and more specifically, it may include 1 to 200-bp 5' upstream and 1 to 200-bp 3' downstream of the marker region, without being limited thereto.

In another aspect, the present invention relates to a composition for diagnosing liver cancer, including a combination of probes each capable of specifically hybridizing to either a polynucleotide including at least 10 contiguous nucleotides, which contains a methylated base of a DNA methylation marker of the combination of DNA methylation markers, or a polynucleotide complementary thereto.

In the present invention, the probe may be methylation-specific, which means that it specifically hybridizes only to a methylated nucleic acid of the methylation marker region. Here, hybridization is usually performed under stringent conditions, for example, a salt concentration of 1 M or less and a temperature of 25°C or higher. For example, conditions of 5X SSPE (750 mM NaCl, 50 mM Na Phosphate, 5 mM EDTA, pH 7.4) and 25 to 30°C may be suitable for methylation-specific probe hybridization.

In the present invention, the probe refers to a hybridization probe, which refers to an oligonucleotide capable of binding sequence-specifically to a complementary strand of a nucleic acid. The methylation-specific probe of the present invention may hybridize to a DNA fragment derived from one individual but not hybridize to a fragment derived from another individual, because methylation exists in nucleic acid fragments derived from two individuals of the same species. In this case, hybridization conditions should be sufficiently stringent that there is a significant difference in hybridization intensity so that hybridization occurs depending on whether methylation is present. It is preferable that the central portion of the probe of the present invention be aligned to the region of the methylation marker. The probe of the present invention may be used in a diagnostic kit or prediction method for determining the presence or absence of liver cancer by detecting the level of DNA methylation.

In another aspect, the present invention relates to

a kit for diagnosing liver cancer, including any one of the above-described compositions.

In the present invention, the kit may include not only the polynucleotide of the present invention, but also one or more other component compositions, solutions or devices suitable for analytical methods. In one embodiment, the kit of the present invention may be a kit including essential elements necessary for performing PCR, and may further include a test tube or other appropriate container, a reaction buffer (various pHs and magnesium concentrations), deoxynucleotides (dNTPs), enzymes such as Taq polymerase and reverse transcriptase, DNase, RNase inhibitor, DEPC-water, and sterile water. In another aspect, the kit of the present invention may be a kit for predicting blood statin concentration, including essential elements necessary for performing DNA chip assay. The DNA chip kit may include a substrate to which the methylation-specific polynucleotide, primer or probe is attached, wherein the substrate may include a nucleic acid corresponding to a quantitative control gene or a fragment thereof.

## Examples

Hereinafter, the present invention will be described in more detail by way of examples. These examples are only intended to illustrate the present invention, and it will be apparent to those skilled in the art that the scope of the present invention is not to be construed as being limited by these examples.

## Example 1. Selection of Liver Cancer-Specific Methylation Regions from TCGA Methylation 450K Array Data

The degree of methylation was determined using Infinium Human Methylation 450k BeadChip array data (UCSC Xena, http://xena.ucsc.edu) from The Cancer Genome Atlas (TCGA). DNA extracted from tissue is converted by bisulfite treatment, and DNA methylation can be determined through modification of cytosine bases. The degree of methylation can be determined for each region, and the beta value, which represents the degree of methylation, was used to select differentially methylated regions between liver cancer tissue and surrounding normal tissue.

TCGA methylation 450k array data were divided into training and test groups as shown in Table 4 below, and marker selection was performed using the training group.

[Table 4]

|  | Solid Tissue Normal | Primary Solid Tumor | Total |
|---|---|---|---|
| **Training** | 35 | 263 | 298 |
| **Test** | 15 | 114 | 129 |
| **Total** | 50 | 377 | 427 |

First, missing values were excluded from about 480k regions. Then, Limma (Linear Models for Microarray Data) software was used to select regions with an FDR value of less than 0.01 and an absolute delta beta value greater than 0.25. Then, sex chromosomes were excluded, and 21,920 hypomethylated regions and 2,277 hypermethylated regions specific to liver cancer were selected.

## Example 2. Next-Generation Sequencing of cfDNA Extracted from Blood (cfMeDIP-Seq)

Blood was collected from 68 liver cirrhosis patients and 283 liver cancer patients, and then subjected to first centrifugation under the conditions of 3,000 rpm, 25°C, and 10 minutes to isolate the plasma portion. Then, the plasma isolated by the first centrifugation was subjected to a second centrifugation under the conditions of 16,000 g, 25°C, and 10 minutes to isolate the plasma supernatant excluding the precipitate. Cell-free DNA was extracted from the separated plasma using the Chemagen DNA kit, and subjected to adaptor ligation using the Truseq Nano DNA HT library prep kit (Illumina), followed by 5mC immunoprecipitation using the antibody of the cfMeDIP kit (Diagnode) at 10 rpm and 4°C for 17 hours. After purification, PCR enrichment was performed using the Truseq Nano DNA HT library prep kit (Illumina) to produce the final library. The produced library was sequenced using Novaseq 6000 (Illumina) in 150 paired-end mode, generating about 100 million reads per sample.

## Example 3. Selection of Liver Cancer-Specific Methylation Regions through cfMeDIP-Seq Data Analysis

Since the methylated cell-free nucleic acid was sequenced in Example 2, the obtained nucleic acid fragment data are in a methylated state and can be aligned to the human reference genome, thereby identifying the methylated regions in the entire human genome. MeDIP-Seq data represent methylated regions, and the normalized values for each 300-bp bin were used to select differentially methylated regions between the liver cancer group and the liver cirrhosis group.

cfMeDIP-Seq data were divided into training, validation, and test groups as shown in Table 5 below. Marker selection and model training were performed using the training group, hyper-parameter tuning was performed using the validation group, and the performance of the constructed model was evaluated using the test group.

[Table 5]

|  | Liver cirrhosis | Liver cancer | Total |
|---|---|---|---|
| **Training** | 40 | 163 | 203 |
| **Validation** | 14 | 55 | 69 |
| **Test** | 14 | 65 | 79 |
| **Total** | 68 | 283 | 351 |

First, adapter trimming and quality trimming of fastq files were performed using Trim Galore (version 0.6.6). Then, nucleic acid fragment data were aligned to the reference genome (hg19) using the BWA (version 0.7.17-r1188) alignment tool, and duplicate PCR fragments were removed using the SAMtools rmdup (version 1.11) tool. Then, nucleic acid fragments with a mapping quality of less than 10 were removed using the SAMtools view (version 1.11) tool. Then, only chr1-22, X, and Y were retained, while the others were discarded. The entire genome was divided into 300-bp bins without overlapping, and then read counts for each 300-bp bin were calculated.

Blacklist regions (Low_mappability_island, centromeric_repeat, etc.) and bins with a total read count of less than 10 across all samples were excluded.

TMM normalized values for each 300-bp bin were generated using the edgeR (Empirical Analysis of Digital Gene Expression Data in R) software.

Finally, using the edgeR software, 2 hypermethylated regions and 2,202 hypomethylated regions specific to liver cancer with an FDR value of less than 0.05 and an absolute value of log2 fold change of more than 2 were selected.

**Example 4. Selection of Major Methylated Regions around CpG**

4-1. Application of cfMeDIP-Seq Data to Markers Selected from TCGA

Input was generated from the cfMeDIP-Seq data obtained in Example 2, focusing on the CpG sites in the regions selected in Example 1. More specifically, CPM normalized values of ±250-bp bins around the CpG sites were used. Only regions with an FDR value of less than 0.05 and an absolute value of log2 fold change of more than were selected using the Wilcoxon rank sum test.

As a result, 1,184 sites showing hypermethylation in the TCGA data and also showing hypermethylation in cfMeDIP-seq were selected.

4-2. Selection of cfMeDIP-Seq Data around CpG Sites

CpGs included in the bins selected in Example 3 were extracted, and CPM normalized values of bins of ±250 bp around the CpGs were used. Only regions with an FDR value of less than 0.05 and an absolute value of the log2 fold change of more than 1 were selected using the Wilcoxon rank sum test.

As a result, 19 hypomethylated regions and 35,400 hypermethylated sites specific to liver cancer were selected.

**Example 5. Construction of Machine Learning Model for Diagnosis of Liver Cancer and Evaluation of Performance Thereof**

5-1. Final Selection of Markers

354 hypermethylated sites selected simultaneously in Examples 4-1 and 4-2 were selected as features, and the detailed list thereof is shown in Table 6 below.

[Table 6]

| chr1 | 119549013 | 119549512 | - |
|---|---|---|---|

(continued)

| chr1 | 146551494 | 146551993 | - |
|------|-----------|-----------|---|
| chr1 | 151812171 | 151812670 | C2CD4D, C2CD4D-AS1 |
| chr1 | 151812185 | 151812684 | C2CD4D, C2CD4D-AS1 |
| chr1 | 151812274 | 151812773 | C2CD4D, C2CD4D-AS1 |
| chr1 | 151812460 | 151812959 | C2CD4D, C2CD4D-AS1 |
| chr1 | 154474923 | 154475422 | SHE, TDRD10 |
| chr1 | 156130576 | 156131075 | SEMA4A |
| chr1 | 156389874 | 156390373 | MIR9-1, MIR9-1HG |
| chr1 | 160951657 | 160952156 | - |
| chr1 | 161275311 | 161275810 | MPZ |
| chr1 | 169396462 | 169396961 | CCDC181 |
| chr1 | 170629820 | 170630319 | PRRX1 |
| chr1 | 170630308 | 170630807 | PRRX1 |
| chr1 | 171810218 | 171810717 | DNM3 |
| chr1 | 171810722 | 171811221 | DNM3 |
| chr1 | 171811049 | 171811548 | DNM3 |
| chr1 | 197882219 | 197882718 | LHX9 |
| chr1 | 203598323 | 203598822 | ATP284 |
| chr1 | 213123425 | 213123924 | VASH2 |
| chr1 | 213123636 | 213124135 | VASH2 |
| chr1 | 213123715 | 213124214 | VASH2 |
| chr1 | 221064450 | 221064949 | - |
| chr1 | 228645797 | 228646296 | H2AW, H2BU1, MIR4666A |
| chr1 | 247171153 | 247171652 | ZNF670-ZNF695, ZNF695 |
| chr2 | 20068452 | 20068951 | LINC00954 |
| chr2 | 25438860 | 25439359 | - |
| chr2 | 45159843 | 45160342 | - |
| chr2 | 45160195 | 45160694 | - |
| chr2 | 45160240 | 45160739 | - |
| chr2 | 45170072 | 45170571 | SIX3 |
| chr2 | 45231217 | 45231716 | - |
| chr2 | 45231532 | 45232031 | - |
| chr2 | 45231641 | 45232140 | |
| chr2 | 45232167 | 45232666 | SIX2 |
| chr2 | 63280819 | 63281318 | OTX1 |
| chr2 | 63280889 | 63281388 | OTX1 |
| chr2 | 63281067 | 63281566 | OTX1 |
| chr2 | 63281133 | 63281632 | OTX1 |
| chr2 | 63281594 | 63282093 | OTX1 |
| chr2 | 63282452 | 63282951 | OTX1 |

(continued)

| chr2 | 63282763 | 63283262 | OTX1 |
|------|----------|----------|------|
| chr2 | 63283717 | 63284216 | OTX1 |
| chr2 | 63283816 | 63284315 | OTX1 |
| chr2 | 63284518 | 63285017 | OTX1 |
| chr2 | 63285799 | 63286298 | - |
| chr2 | 74425262 | 74425761 | MTHFD2 |
| chr2 | 74425330 | 74425829 | MTHFD2 |
| chr2 | 74781846 | 74782345 | DOK1, LOXL3 |
| chr2 | 105470311 | 105470810 | PANTR1, POU3F3 |
| chr2 | 119067387 | 119067886 | - |
| chr2 | 119067503 | 119068002 | - |
| chr2 | 157177686 | 157178185 | - |
| chr2 | 157178481 | 157178980 | - |
| chr2 | 157178639 | 157179138 | - |
| chr2 | 160761163 | 160761662 | LY75, LY75-CD302 |
| chr2 | 176987215 | 176987714 | HOXD9 |
| chr2 | 177003485 | 177003984 | HOXD-AS2 |
| chr2 | 177003497 | 177003996 | HOXD-AS2 |
| chr2 | 200331667 | 200332166 | SATB2, SATB2-AS1 |
| chr2 | 200331727 | 200332226 | SATB2, SATB2-AS1 |
| chr2 | 200331775 | 200332274 | SATB2, SATB2-AS1 |
| chr2 | 200333751 | 200334250 | SATB2, SATB2-AS1 |
| chr2 | 200334851 | 200335350 | SATB2, SATB2-AS1 |
| chr2 | 238583254 | 238583753 | LRRFIP1 |
| chr3 | 38080675 | 38081174 | DLEC1 |
| chr3 | 101497626 | 101498125 | NXPE3 |
| chr3 | 101497730 | 101498229 | NXPE3 |
| chr3 | 101497732 | 101498231 | NXPE3 |
| chr3 | 138658771 | 138659270 | - |
| chr3 | 138662065 | 138662564 | FOXL2NB |
| chr3 | 138662980 | 138663479 | FOXL2, FOXL2NB |
| chr3 | 142837745 | 142838244 | CHST2 |
| chr3 | 147098318 | 147098817 | - |
| chr3 | 147098335 | 147098834 | - |
| chr3 | 147105760 | 147106259 | ZIC4 |
| chr3 | 147136654 | 147137153 | - |
| chr3 | 169529777 | 169530276 | LRRC34 |
| chr3 | 169529787 | 169530286 | LRRC34 |
| chr3 | 179168510 | 179169009 | GNB4 |
| chr3 | 179168548 | 179169047 | GNB4 |

(continued)

| chr3 | 183145282 | 183145781 | MCF2L2 |
|------|-----------|-----------|--------|
| chr3 | 186490406 | 186490905 | - |
| chr4 | 785994 | 786493 | CPLX1 |
| chr4 | 41868836 | 41869335 | |
| chr4 | 41880497 | 41880996 | - |
| chr4 | 41881913 | 41882412 | - |
| chr4 | 41882330 | 41882829 | - |
| chr4 | 76555297 | 76555796 | CDKL2 |
| chr4 | 76555384 | 76555883 | CDKL2 |
| chr4 | 76555522 | 76556021 | CDKL2 |
| chr4 | 76555527 | 76556026 | CDKL2 |
| chr4 | 76555532 | 76556031 | CDKL2 |
| chr5 | 7849953 | 7850452 | C5orf49 |
| chr5 | 7850188 | 7850687 | C5orf49 |
| chr5 | 32713473 | 32713972 | NPR3 |
| chr5 | 40680887 | 40681386 | PTGER4 |
| chr5 | 40681643 | 40682142 | PTGER4 |
| chr5 | 42950942 | 42951441 | - |
| chr5 | 42951863 | 42952362 | - |
| chr5 | 42992524 | 42993023 | - |
| chr5 | 43017435 | 43017934 | LOC648987 |
| chr5 | 43018193 | 43018692 | LOC648987 |
| chr5 | 54516555 | 54517054 | MCIDAS |
| chr5 | 94955506 | 94956005 | GPR150 |
| chr5 | 112073100 | 112073599 | APC |
| chr5 | 112073123 | 112073622 | APC |
| chr5 | 112073148 | 112073647 | APC |
| chr5 | 112073156 | 112073655 | APC |
| chr5 | 112073176 | 112073675 | APC |
| chr5 | 112073188 | 112073687 | APC |
| chr5 | 139047755 | 139048254 | CXXCS |
| chr5 | 139047856 | 139048355 | CXXC5 |
| chr5 | 169064201 | 169064700 | DOCK2 |
| chr5 | 170736027 | 170736526 | TLX3 |
| chr6 | 1624861 | 1625360 | GMDS |
| chr6 | 3228983 | 3229482 | TUBB2B |
| chr6 | 5026074 | 5026573 | - |
| chr6 | 5026185 | 5026684 | - |
| chr6 | 10425398 | 10425897 | - |
| chr6 | 10425849 | 10426348 | - |

(continued)

| chr6 | 26235004 | 26235503 | H1-3 |
|------|----------|----------|------|
| chr6 | 26240532 | 26241031 | H4C6 |
| chr6 | 26240670 | 26241169 | H4C6 |
| chr6 | 26250494 | 26250993 | H2BC9, H3C7, H4C7 |
| chr6 | 26250669 | 26251168 | H2BC9. H3C7, H4C7 |
| chr6 | 26250686 | 26251185 | H2BC9, H3C7, H4C7 |
| chr6 | 26251649 | 26252148 | H28C9, H3C7, H4C7 |
| chr6 | 26252015 | 26252514 | H2BC9, H3C7, H4C7 |
| chr6 | 26271466 | 26271965 | H2BC10, H3C8 |
| chr6 | 26271468 | 26271967 | H2BC10, H3C8 |
| chr6 | 26271566 | 26272065 | H2BC10, H3C8 |
| chr6 | 26271577 | 26272076 | H2BC10, H3C8 |
| chr6 | 26550760 | 26551259 | - |
| chr6 | 26614399 | 26614898 | - |
| chr6 | 27462967 | 27463466 | - |
| chr6 | 27858387 | 27858886 | H2BC17, H3C12 |
| chr6 | 28411037 | 28411536 | ZSCAN23 |
| chr6 | 42738717 | 42739216 | - |
| chr6 | 42738799 | 42739298 | - |
| chr6 | 100911437 | 100911936 | SIM1 |
| chr6 | 100912656 | 100913155 | SIM1 |
| chr6 | 100912690 | 100913189 | SIM1 |
| chr6 | 100912696 | 100913195 | SIM1 |
| chr6 | 100915517 | 100916016 | SIM1 |
| chr6 | 108488085 | 108488584 | NR2E1 |
| chr6 | 108490645 | 108491144 | NR2E1 |
| chr6 | 108495615 | 108496114 | NR2E1 |
| chr6 | 108495735 | 108496234 | NR2E1 |
| chr6 | 133561851 | 133562350 | EYA4 |
| chr6 | 133562216 | 133562715 | EYA4 |
| chr6 | 133562220 | 133562719 | EVA4 |
| chr6 | 133562225 | 133562724 | EYA4 |
| chr6 | 133562229 | 133562728 | EYA4 |
| chr6 | 133562235 | 133562734 | EYA4 |
| chr6 | 133562242 | 133562741 | EYA4 |
| chr6 | 133562244 | 133562743 | EYA4 |
| chr7 | 27204478 | 27204977 | HOXA10-AS, HOXA10-HOXA9, HOXA9 |
| chr7 | 27204731 | 27205230 | HOXA10-AS, HOXA10-HOXA9, HOXA9 |
| chr7 | 27204967 | 27205466 | HOXA10-AS, HOXA10-HOXA9, HOXA9 |
| chr7 | 27213734 | 27214233 | HOXA10, HOXA10-HOXA9, MIR1968 |

(continued)

| chr7 | 27213793 | 27214292 | HOXA10, HOXA10-HOXA9, MIR1968 |
|------|----------|----------|-------------------------------|
| chr7 | 27213806 | 27214305 | HOXA10, HOXA10-HOXA9, MIR1968 |
| chr7 | 27232587 | 27233086 | - |
| chr7 | 27232823 | 27233322 | - |
| chr7 | 27232891 | 27233390 | - |
| chr7 | 27245292 | 27245791 | HOTTIP |
| chr7 | 27252291 | 27252790 | - |
| chr7 | 27284539 | 27285038 | EVX1 |
| chr7 | 27291096 | 27291595 | - |
| chr7 | 28996389 | 28996888 | TRIL |
| chr7 | 28996652 | 28997151 | TRIL |
| chr7 | 28996923 | 28997422 | TRIL |
| chr7 | 28997235 | 28997734 | TRIL |
| chr7 | 28997616 | 28998115 | TRIL |
| chr7 | 28997828 | 28998327 | TRIL |
| chr7 | 76828635 | 76829134 | CCDC146, FGL2 |
| chr7 | 96636366 | 96636865 | DLX6, DLX6-AS1 |
| chr7 | 96636496 | 96636995 | DLX6, DLX6-AS1 |
| chr7 | 96651031 | 96651530 | DLXS |
| chr7 | 96651865 | 96652364 | DLXS |
| chr7 | 96651873 | 96652372 | DLX5 |
| chr7 | 117119174 | 117119673 | CFTR |
| chr7 | 117119351 | 117119850 | CFTR |
| chr7 | 117119361 | 117119860 | CFTR |
| chr7 | 117119387 | 117119886 | CFTR |
| chr7 | 117119688 | 117120187 | CFTR |
| chr7 | 134143656 | 134144155 | AKR1B1 |
| chr7 | 134143669 | 134144168 | AKR1B1 |
| chr7 | 134143786 | 134144285 | AKR181 |
| chr7 | 143042491 | 143042990 | CLCN1 |
| chr7 | 143042548 | 143043047 | CLCN1 |
| chr7 | 151329758 | 151330257 | PRKAG2 |
| chr8 | 11540157 | 11540656 | GATA4 |
| chr8 | 41424092 | 41424591 | - |
| chr8 | 49292435 | 49292934 | - |
| chr8 | 53851934 | 53852433 | NPBWR1 |
| chr8 | 57069657 | 57070156 | - |
| chr8 | 59058004 | 59058503 | FAM1108 |
| chr8 | 67873093 | 67873592 | TCF24 |
| chr8 | 67873226 | 67873725 | TCF24 |

(continued)

| | | | |
|---|---|---|---|
| chr8 | 67873549 | 67874048 | TCF24 |
| chr8 | 67873928 | 67874427 | TCF24 |
| chr8 | 67874116 | 67874615 | TCF24 |
| chr8 | 67874783 | 67875282 | TCF24 |
| chr8 | 70981789 | 70982288 | PRDM14 |
| chr8 | 86350318 | 86350817 | CA13, CA3 |
| chr8 | 86350331 | 86350830 | CA13, CA3 |
| chr8 | 98289898 | 98290397 | TSPYL5 |
| chr8 | 99951797 | 99952296 | OSR2, STK3 |
| chr8 | 99959473 | 99959972 | OSR2, STK3 |
| chr8 | 99959898 | 99960397 | OSR2 |
| chr8 | 99961295 | 99961794 | OSR2 |
| chr8 | 99961624 | 99962123 | OSR2 |
| chr8 | 102504197 | 102504696 | GRHL2 |
| chr8 | 102504251 | 102504750 | GRHL2 |
| chr8 | 102504314 | 102504813 | GRHL2 |
| chr8 | 102505306 | 102505805 | GRHL2 |
| chr8 | 104512833 | 104513332 | RIMS2 |
| chr9 | 110228019 | 110228518 | - |
| chr10 | 8094284 | 8094783 | GATA3, GATA3-AS1 |
| chr10 | 16562220 | 16562719 | C1QL3 |
| chr10 | 17271679 | 17272178 | VIM |
| chr10 | 17271694 | 17272193 | VIM |
| chr10 | 17271744 | 17272243 | VIM |
| chr10 | 17271867 | 17272366 | VIM |
| chr10 | 22541774 | 22542273 | LOC100130992 |
| chr10 | 22625215 | 22625714 | - |
| chr10 | 22765590 | 22766089 - | |
| chr10 | 43697758 | 43698257 | RASGEF1A |
| chr10 | 93647050 | 93647549 | - |
| chr1 0 | 94834332 | 94834831 | CYP26A1 |
| chr10 | 94834513 | 94835012 | CYP26A1 |
| chr10 | 102894793 | 102895292 | TLX1, TLX1NB |
| chr10 | 104000581 | 104001080 | GBF1, PITX3 |
| chr11 | 13689872 | 13690371 | FAR1 |
| chr11 | 13689910 | 13690409 | FAR1 |
| chr11 | 13690452 | 13690951 | FAR1 |
| chr11 | 31826324 | 31826823 | PAX6 |
| chr1 1 | 69517049 | 69517548 | FGF1 9 |
| chr11 | 69517221 | 69517720 | FGF19 |

(continued)

| chr11 | 69517591 | 69518090 | FGF19 |
|---|---|---|---|
| chr11 | 69517753 | 69518252 | FGF19 |
| chr11 | 69517947 | 69518446 | FGF19 |
| chr12 | 21810029 | 21810528 | LDHB |
| chr12 | 21810380 | 21810879 | LDHB |
| chr12 | 21810558 | 21811057 | LDHB |
| chr12 | 48206598 | 48207097 | HDAC7 |
| chr12 | 58021319 | 58021818 | B4GALNT1 |
| chr12 | 58021463 | 58021962 | 84GALNT1 |
| chr12 | 81102236 | 81102735 | MYF6 |
| chr12 | 95941619 | 95942118 | USP44 |
| chr12 | 95941738 | 95942237 | USP44 |
| chr12 | 95942511 | 95943010 | USP44 |
| chr12 | 95942657 | 95943156 | USP44 |
| chr12 | 95942714 | 95943213 | USP44 |
| chr12 | 115102476 | 115102975 | - |
| chr13 | 45149778 | 45150277 | TSC22D1, TSC22D1-AS1 |
| chr13 | 45150012 | 45150511 | TSC22D1, TSC22D1-AS1 |
| chr1 3 | 100627091 | 100627590 | ZIC5 |
| chr1 3 | 100641159 | 100641658 | - |
| chr13 | 100641396 | 100641895 | - |
| chr13 | 107186620 | 107187119 | EFNB2 |
| chr13 | 107186927 | 107187426 | EFNB2 |
| chr14 | 51027611 | 51028110 | ATL1 |
| chr14 | 54422525 | 54423024 | BMP4 |
| chr14 | 54423183 | 54423682 | BMP4 |
| chr14 | 61108957 | 61109456 | - |
| chr14 | 100632700 | 100633199 | - |
| chr15 | 33009281 | 33009780 | GREM1 |
| chr15 | 41805280 | 41805779 | LTK |
| chr15 | 55880644 | 55881143 | PYGO1 |
| chr15 | 65186056 | 65186555 | - |
| chr15 | 68260324 | 68260823 | - |
| chr15 | 69087559 | 69088058 | ANP32A |
| chr15 | 99193679 | 99194178 | IGF1R |
| chr15 | 99193743 | 99194242 | IGF1R |
| chr15 | 101459033 | 101459532 | LRRK1 |
| chr16 | 21170817 | 21171316 | DNAH3, TMEM159 |
| chr17 | 4981360 | 4981859 | ZFP3 |
| chr17 | 4981573 | 4982072 | ZFP3 |

(continued)

| chr17 | 29297898 | 29298397 | DPRXP4, RNF135 |
|-------|----------|----------|----------------|
| chr17 | 29297934 | 29298433 | DPRXP4, RNF135 |
| chr17 | 29298102 | 29298601 | DPRXP4, RNF135 |
| chr17 | 42030229 | 42030728 | PYY |
| chr17 | 43338973 | 43339472 | MAP3K14-AS1, SPATA32 |
| chr17 | 43339078 | 43339577 | MAP3K14-AS1, SPATA32 |
| chr17 | 43339247 | 43339746 | MAP3K14-AS1, SPATA32 |
| chr17 | 43339262 | 43339761 | MAP3K14-AS1, SPATA32 |
| chr17 | 46655579 | 46656078 | HOXB3, HOXB4 |
| chr17 | 48636396 | 48636895 | CACNA1G, CACNA1G-AS1 |
| chr17 | 59529066 | 59529565 | TBX4 |
| chr17 | 59529236 | 59529735 | TBX4 |
| chr17 | 59534597 | 59535096 | TBX4 |
| chr17 | 59534748 | 59535247 | TBX4 |
| chr17 | 62777398 | 62777897 | ARHGAP27P1, PLEKHM1P1 |
| chr17 | 79480858 | 79481357 | ACTG1 |
| chr17 | 80291438 | 80291937 | SECTM1 |
| chr18 | 32847001 | 32847500 | ZNF397, ZSCAN30 |
| chr18 | 55019849 | 55020348 | ST8SIA3 |
| chr19 | 12305604 | 12306103 | - |
| chr19 | 12305619 | 12306118 | - |
| chr19 | 12305886 | 12306385 | - |
| chr19 | 12305948 | 12306447 | - |
| chr19 | 13209731 | 132102,30 | LYL1 |
| chr19 | 36736022 | 36736521 | - |
| chr19 | 38182805 | 38183304 | ZNF781 |
| chr19 | 38754889 | 38755388 | SPINT2 |
| chr19 | 41316817 | 41317316 | - |
| chr19 | 42901057 | 42901556 | LIPE-AS1 |
| chr19 | 50553817 | 50554316 | LOC400710 |
| chr19 | 50554030 | 50554529 | LOC400710 |
| chr19 | 50554201 | 50554700 | LOC400710 |
| chr19 | 52207103 | 52207602 | SPACA6 |
| chr19 | 57018819 | 57019318 | ZNF471 |
| chr19 | 58220045 | 58220544 | ZNF154, ZNF776 |
| chr19 | 58220120 | 58220619 | ZNF154, ZNF776 |
| chr20 | 1784026 | 1784525 | - |
| chr20 | 30778049 | 30778548 | TSPY26P |
| chr20 | 37433979 | 37434478 | PPP1R16B |
| chr20 | 50720658 | 50721157 | ZFP64 |

(continued)

| chr20 | 50721063 | 50721562 | ZFP64 |
|-------|----------|----------|-------|

5-2. Construction of Machine Learning Model

CPM normalized values of binds of $\pm 250$ bp around the selected 354 CpGs were used as input. An artificial neural network model that distinguishes between liver cirrhosis and liver cancer patients was constructed using the input features. The artificial neural network algorithm used for training was a CNN model.

As shown in Table 5 in Example 3, the entire sample was divided into training, validation, and test data sets. The training data set was used for model training, the validation data set was used for hyper-parameter tuning, and the test data set was used for final model performance evaluation.

The CNN model structure consists of, in order, convolution layer -> pooling layer -> fully connected layer, and the pooling layer is always inserted after the convolution layer. Thus, the number of the convolution layers and the number of the fully connected layers were determined through a hyper-parameter tuning process, and when training the model, the training was conducted in the direction of minimizing the loss function of Equation 1.

Hyper-parameter tuning is a process of optimizing the values of various parameters (n_estimators, criterion, max_features, max_depth, and min_samples_leaf values) constituting the RandomForest model. The hyper-parameter tuning process was performed using the Bayesian optimization technique, and when validation loss started to increase compared to training loss, the model was determined to be over-fit, and training of the model was stopped.

The performance of various models obtained through hyper-parameter tuning was compared using the validation data set, and then the model having the best validation data set performance was determined to be the optimal model. Data were input into the selected model, and the DPI value was obtained using the sigmoid function of Equation 2 in the output layer. Final performance evaluation was performed using the test data set, and the presence or absence of cancer was predicted using the calculated predicted probability (DPI value). If the calculated probability value exceeded 0.5, liver cancer was determined to be present.

5-3. Evaluation of Performance of Machine Learning Model

As in Table 7 below and FIG. 2, it was confirmed that, when the model constructed in Example 5-2 was used, the accuracies were 0.93, 0.93, and 0.84 in the training, validation, and test groups, respectively, and that the AUC values, which are the results of ROC analysis, were 0.96, 0.97, and 0.90 in the training, validation, and test groups, respectively.

[Table 7]

|            | Accuracy | AUC  |
|------------|----------|------|
| Training   | 0.93     | 0.96 |
| Validation | 0.93     | 0.97 |
| Test       | 0.84     | 0.90 |

**Example 8. Selection of Liver Cancer-Specific Methylation Regions from TCGA Methylation 450K Array Data**

In addition, in order to find the minimum combination of liver cancer-specific methylation regions, the degree of methylation was measured using Infinium Human Methylation 450k BeadChip array data (UCSC Xena, http://xena.ucsc. edu) from The Cancer Genome Atlas (TCGA). DNA extracted from tissue is converted by bisulfite treatment, and DNA methylation can be determined through modification of cytosine bases. The degree of methylation can be determined for each region, and the beta value, which represents the degree of methylation, was used to select differentially methylated regions between liver cancer tissue and surrounding normal tissue.

TCGA methylation 450k array data are shown in Table 8 below.

[Table 8]

|      | Primary Solid Tumor | Solid Tissue Normal | Total |
|------|---------------------|---------------------|-------|
| LIHC | 377                 | 50                  | 427   |

First, missing values were excluded from about 480k sites, and then the methylation score was used and calculated to select significantly hypermethylated regions. The methylation score is the sum of the beta values of all selected CpGs for each sample.

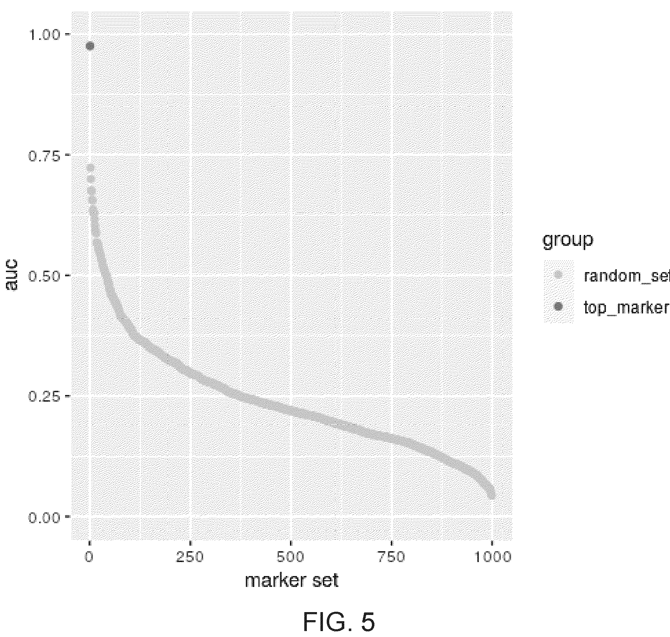

Sets of CpG sites were constructed by randomly sampling 20 CpG sites 1000 times with replacement, and then each set of CpG sites was used to calculate the methylation scores of 427 liver cancer tissues and surrounding normal tissues (FIG. 5).

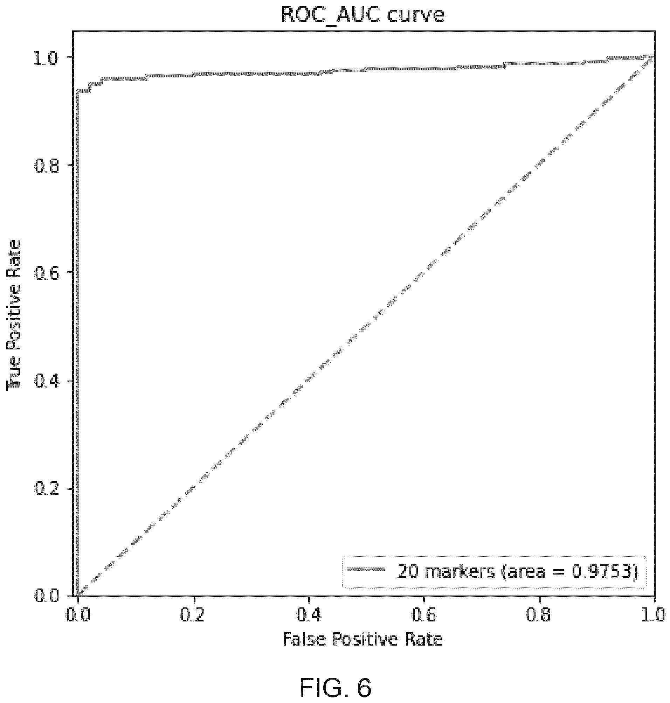

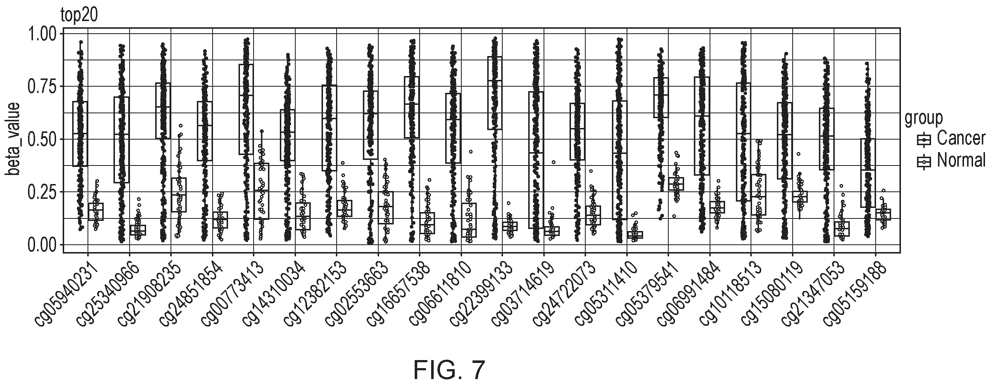

The AUC for distinguishing between liver cancer tissues and surrounding normal tissues was calculated using the methylation score, and a set of CpG sites with the highest AUC was selected. As a result, 20 hypermethylated regions with an AUC of 0.975 as shown in Table 9 below were selected (FIGS. 6 and 7).

[Table 9]

| Chromosome | Start | End | Gene | Illumina ProbID | Added marker |
|---|---|---|---|---|---|
| chr1 | 59042275 | 59042276 | TACSTD2 | cg24851854 | O |
| chr1 | 119532189 | 119532190 | TBX15 | cg05940231 | X |
| chr1 | 119532195 | 119532196 | TBX15 | cg25340966 | X |
| chr1 | 146551744 | 146551745 | - | cg21908235 | X |
| chr2 | 45160445 | 45160446 | - | cg03714619 | X |
| chr2 | 208989248 | 208989249 | CRYGD / LOC100507443 | cg22399133 | O |
| chr4 | 41882163 | 41882164 | LINC00682 | cg24722073 | X |
| chr6 | 1624978 | 1624979 | GMDS | cg21347053 | X |
| chr6 | 26240579 | 26240580 | H4C6 | cg05159188 | X |
| chr6 | 26252265 | 26252266 | H2BC9 | cg15080119 | X |
| chr7 | 27225523 | 27225524 | HOXA11 / HOXA11-AS | cg05311410 | O |
| chr7 | 27252541 | 27252542 | - | cg05379541 | X |
| chr8 | 11540407 | 11540408 | GATA4 | cg06991484 | X |
| chr10 | 77168431 | 77168432 | ZNF503-AS2 | cg00773413 | O |
| chr14 | 54423433 | 54423434 | BMP4 | cg14310034 | X |
| chr14 | 100632950 | 100632951 | - | cg10118513 | X |
| chr15 | 58357204 | 58357205 | ALDH1A2 | cg12382153 | O |
| chr17 | 80291775 | 80291776 | SECTM1 | cg02553663 | X |
| chr18 | 32847566 | 32847567 | ZSCAN30 | cg16657538 | O |
| chr19 | 41317067 | 41317068 | - | cg06611810 | X |

A combination of 360 methylation markers obtained by combining both markers is shown in Table 10 below.

[Table 10]

| chr | start | end | gene |
|---|---|---|---|
| chr1 | 46632446 | 46632945 | PIK3R3 |
| chr1 | 46632621 | 46633120 | PIK3R3 |
| chr1 | 47697715 | 47698214 | STIL, TAL1 |
| chr1 | 47908984 | 47909483 | - |
| chr1 | 47909931 | 47910430 | - |
| chr1 | 47910206 | 47910705 | - |
| chr1 | 47910593 | 47911092 | - |
| chr1 | 48058627 | 48059126 | - |
| chr1 | 48058711 | 48059210 | - |

(continued)

| chr | start | end | gene |
|------|-----------|-----------|---------------------|
| chr1 | 67772878 | 67773377 | IL12RB2 |
| chr1 | 87617460 | 87617959 | LINC01140 |
| chr1 | 91192216 | 91192715 | - |
| chr1 | 110610649 | 110611148 | ALX3 |
| chr1 | 119522605 | 119523104 | TBX15 |
| chr1 | 119526861 | 119527360 | TBX15 |
| chr1 | 119526906 | 119527405 | TBX15 |
| chr1 | 119527634 | 119528133 | TBX15 |
| chr1 | 119529680 | 119530179 | TBX15 |
| chr1 | 119531806 | 119532305 | TBX15 |
| chr1 | 119531866 | 119532365 | TBX15 |
| chr1 | 119531939 | 119532438 | TBX15 |
| chr1 | 119531945 | 119532444 | TBX15 |
| chr1 | 119532523 | 119533022 | TBX15 |
| chr1 | 119532675 | 119533174 | TBX15 |
| chr1 | 119542807 | 119543306 | - |
| chr1 | 119542966 | 119543465 | - |
| chr1 | 119543086 | 119543585 | - |
| chr1 | 119548277 | 119548776 | - |
| chr1 | 119548575 | 119549074 | - |
| chr1 | 119548602 | 119549101 | - |
| chr1 | 119548895 | 119549394 | - |
| chr1 | 119549013 | 119549512 | - |
| chr1 | 151812171 | 151812670 | C2CD4D, C2CD4D-AS1 |
| chr1 | 151812185 | 151812684 | C2CD4D, C2CD4D-AS1 |
| chr1 | 151812274 | 151812773 | C2CD4D, C2CD4D-AS1 |
| chr1 | 151812460 | 151812959 | C2CD4D, C2CD4D-AS1 |
| chr1 | 154474923 | 154475422 | SHE, TDRD10 |
| chr1 | 156130576 | 156131075 | SEMA4A |
| chr1 | 156389874 | 156390373 | MIR9-1, MIR9-1HG |
| chr1 | 160951657 | 160952156 | - |
| chr1 | 161275311 | 161275810 | MPZ |
| chr1 | 169396462 | 169396961 | CCDC181 |
| chr1 | 170629820 | 170630319 | PRRX1 |
| chr1 | 170630308 | 170630807 | PRRX1 |
| chr1 | 171810218 | 171810717 | DNM3 |
| chr1 | 171810722 | 171811221 | DNM3 |
| chr1 | 171811049 | 171811548 | DNM3 |
| chr1 | 197882219 | 197882718 | LHX9 |

(continued)

| chr | start | end | gene |
|---|---|---|---|
| chr1 | 203598323 | 203598822 | ATP2B4 |
| chr1 | 213123425 | 213123924 | VASH2 |
| chr1 | 213123636 | 213124135 | VASH2 |
| chr1 | 213123715 | 213124214 | VASH2 |
| chr1 | 221064450 | 221064949 | - |
| chr1 | 228645797 | 228646296 | H2AW, H2BU1, MIR4666A |
| chr1 | 247171153 | 247171652 | ZNF670-ZNF695, ZNF695 |
| chr1 | 119531794 | 119532293 | TBX15 |
| chr1 | 146551494 | 146551993 | - |
| chr1 | 59042275 | 59042276 | TACSTD2 |
| chr10 | 8094284 | 8094783 | GATA3, GATA3-AS1 |
| chr10 | 16562220 | 16562719 | C1QL3 |
| chr10 | 17271679 | 17272178 | VIM |
| chr10 | 17271694 | 17272193 | VIM |
| chr10 | 17271744 | 17272243 | VIM |
| chr10 | 17271867 | 17272366 | VIM |
| chr10 | 22541774 | 22542273 | LOC100130992 |
| chr10 | 22625215 | 22625714 | - |
| chr10 | 22765590 | 22766089 | - |
| chr10 | 43697758 | 43698257 | RASGEF1A |
| chr10 | 93647050 | 93647549 | - |
| chr10 | 94834332 | 94834831 | CYP26A1 |
| chr10 | 94834513 | 94835012 | CYP26A1 |
| chr10 | 102894793 | 102895292 | TLX1, TLX1NB |
| chr10 | 104000581 | 104001080 | GBF1, PITX3 |
| chr10 | 77168431 | 77168432 | ZNF503-AS2 |
| chr11 | 13689872 | 13690371 | FAR1 |
| chr11 | 13689910 | 13690409 | FAR1 |
| chr11 | 13690452 | 13690951 | FAR1 |
| chr11 | 31826324 | 31826823 | PAX6 |
| chr11 | 69517049 | 69517548 | FGF19 |
| chr11 | 69517221 | 69517720 | FGF19 |
| chr11 | 69517591 | 69518090 | FGF19 |
| chr11 | 69517753 | 69518252 | FGF19 |
| chr11 | 69517947 | 69518446 | FGF19 |
| chr12 | 21810029 | 21810528 | LDHB |
| chr12 | 21810380 | 21810879 | LDHB |
| chr12 | 21810558 | 21811057 | LDHB |
| chr12 | 48206598 | 48207097 | HDAC7 |

(continued)

| chr | start | end | gene |
|---|---|---|---|
| chr12 | 58021319 | 58021818 | B4GALNT1 |
| chr12 | 58021463 | 58021962 | B4GALNT1 |
| chr12 | 81102236 | 81102735 | MYF6 |
| chr12 | 95941619 | 95942118 | USP44 |
| chr12 | 95941738 | 95942237 | USP44 |
| chr12 | 95942511 | 95943010 | USP44 |
| chr12 | 95942657 | 95943156 | USP44 |
| chr12 | 95942714 | 95943213 | USP44 |
| chr12 | 115102476 | 115102975 | - |
| chr13 | 45149778 | 45150277 | TSC22D1, TSC22D1-AS1 |
| chr13 | 45150012 | 45150511 | TSC22D1, TSC22D1-AS1 |
| chr13 | 100627091 | 100627590 | ZIC5 |
| chr13 | 100641159 | 100641658 | - |
| chr13 | 100641396 | 100641895 | - |
| chr13 | 107186620 | 107187119 | EFNB2 |
| chr13 | 107186927 | 107187426 | EFNB2 |
| chr14 | 51027611 | 51028110 | ATL1 |
| chr14 | 54422525 | 54423024 | BMP4 |
| chr14 | 61108957 | 61109456 | - |
| chr14 | 54423183 | 54423682 | BMP4 |
| chr14 | 100632700 | 100633199 | - |
| chr15 | 33009281 | 33009780 | GREM1 |
| chr15 | 41805280 | 41805779 | LTK |
| chr15 | 55880644 | 55881143 | PYGO1 |
| chr15 | 65186056 | 65186555 | - |
| chr15 | 68260324 | 68260823 | - |
| chr15 | 69087559 | 69088058 | ANP32A |
| chr15 | 99193679 | 99194178 | IGF1R |
| chr15 | 99193743 | 99194242 | IGF1R |
| chr15 | 101459033 | 101459532 | LRRK1 |
| chr15 | 58357204 | 58357205 | ALDH1A2 |
| chr16 | 21170817 | 21171316 | DNAH3, TMEM159 |
| chr17 | 4981360 | 4981859 | ZFP3 |
| chr17 | 4981573 | 4982072 | ZFP3 |
| chr17 | 29297898 | 29298397 | DPRXP4, RNF135 |
| chr17 | 29297934 | 29298433 | DPRXP4, RNF135 |
| chr17 | 29298102 | 29298601 | DPRXP4, RNF135 |
| chr17 | 42030229 | 42030728 | PYY |
| chr17 | 43338973 | 43339472 | MAP3K14-AS1, SPATA32 |

(continued)

| chr | start | end | gene |
|---|---|---|---|
| chr17 | 43339078 | 43339577 | MAP3K14-AS1, SPATA32 |
| chr17 | 43339247 | 43339746 | MAP3K14-AS1, SPATA32 |
| chr17 | 43339262 | 43339761 | MAP3K14-AS1, SPATA32 |
| chr17 | 46655579 | 46656078 | HOXB3, HOXB4 |
| chr17 | 48636396 | 48636895 | CACNA1G, CACNA1G-AS1 |
| chr17 | 59529066 | 59529565 | TBX4 |
| chr17 | 59529236 | 59529735 | TBX4 |
| chr17 | 59534597 | 59535096 | TBX4 |
| chr17 | 59534748 | 59535247 | TBX4 |
| chr17 | 62777398 | 62777897 | ARHGAP27P1, PLEKHM1P1 |
| chr17 | 79480858 | 79481357 | ACTG1 |
| chr17 | 80291438 | 80291937 | SECTM1 |
| chr18 | 32847001 | 32847500 | ZNF397, ZSCAN30 |
| chr18 | 55019849 | 55020348 | ST8SIA3 |
| chr18 | 32847566 | 32847567 | ZSCAN30 |
| chr19 | 12305604 | 12306103 | - |
| chr19 | 12305619 | 12306118 | - |
| chr19 | 12305886 | 12306385 | - |
| chr19 | 12305948 | 12306447 | - |
| chr19 | 13209731 | 13210230 | LYL1 |
| chr19 | 36736022 | 36736521 | - |
| chr19 | 38182805 | 38183304 | ZNF781 |
| chr19 | 38754889 | 38755388 | SPINT2 |
| chr19 | 42901057 | 42901556 | LIPE-AS1 |
| chr19 | 50553817 | 50554316 | LOC400710 |
| chr19 | 50554030 | 50554529 | LOC400710 |
| chr19 | 50554201 | 50554700 | LOC400710 |
| chr19 | 52207103 | 52207602 | SPACA6 |
| chr19 | 57018819 | 57019318 | ZNF471 |
| chr19 | 58220045 | 58220544 | ZNF154, ZNF776 |
| chr19 | 58220120 | 58220619 | ZNF154, ZNF776 |
| chr19 | 41316817 | 41317316 | - |
| chr2 | 20068452 | 20068951 | LINC00954 |
| chr2 | 25438860 | 25439359 | - |
| chr2 | 45159843 | 45160342 | - |
| chr2 | 45160195 | 45160694 | - |
| chr2 | 45170072 | 45170571 | SIX3 |
| chr2 | 45231217 | 45231716 | - |
| chr2 | 45231532 | 45232031 | - |

(continued)

| chr | start | end | gene |
|---|---|---|---|
| chr2 | 45231641 | 45232140 | - |
| chr2 | 45232167 | 45232666 | SIX2 |
| chr2 | 63280819 | 63281318 | OTX1 |
| chr2 | 63280889 | 63281388 | OTX1 |
| chr2 | 63281067 | 63281566 | OTX1 |
| chr2 | 63281133 | 63281632 | OTX1 |
| chr2 | 63281594 | 63282093 | OTX1 |
| chr2 | 63282452 | 63282951 | OTX1 |
| chr2 | 63282763 | 63283262 | OTX1 |
| chr2 | 63283717 | 63284216 | OTX1 |
| chr2 | 63283816 | 63284315 | OTX1 |
| chr2 | 63284518 | 63285017 | OTX1 |
| chr2 | 63285799 | 63286298 | - |
| chr2 | 74425262 | 74425761 | MTHFD2 |
| chr2 | 74425330 | 74425829 | MTHFD2 |
| chr2 | 74781846 | 74782345 | DOK1, LOXL3 |
| chr2 | 105470311 | 105470810 | PANTR1, POU3F3 |
| chr2 | 119067387 | 119067886 | - |
| chr2 | 119067503 | 119068002 | - |
| chr2 | 157177686 | 157178185 | - |
| chr2 | 157178481 | 157178980 | - |
| chr2 | 157178639 | 157179138 | - |
| chr2 | 160761163 | 160761662 | LY75, LY75-CD302 |
| chr2 | 176987215 | 176987714 | HOXD9 |
| chr2 | 177003485 | 177003984 | HOXD-AS2 |
| chr2 | 177003497 | 177003996 | HOXD-AS2 |
| chr2 | 200331667 | 200332166 | SATB2, SATB2-AS1 |
| chr2 | 200331727 | 200332226 | SATB2, SATB2-AS1 |
| chr2 | 200331775 | 200332274 | SATB2, SATB2-AS1 |
| chr2 | 200333751 | 200334250 | SATB2, SATB2-AS1 |
| chr2 | 200334851 | 200335350 | SATB2, SATB2-AS1 |
| chr2 | 238583254 | 238583753 | LRRFIP1 |
| chr2 | 45160240 | 45160739 | - |
| chr2 | 208989248 | 208989249 | CRYGD / LOC100507443 |
| chr20 | 1784026 | 1784525 | - |
| chr20 | 30778049 | 30778548 | TSPY26P |
| chr20 | 37433979 | 37434478 | PPP1R16B |
| chr20 | 50720658 | 50721157 | ZFP64 |
| chr20 | 50721063 | 50721562 | ZFP64 |

(continued)

| chr | start | end | gene |
|------|------------|------------|----------------|
| chr3 | 38080675 | 38081174 | DLEC1 |
| chr3 | 101497626 | 101498125 | NXPE3 |
| chr3 | 101497730 | 101498229 | NXPE3 |
| chr3 | 101497732 | 101498231 | NXPE3 |
| chr3 | 138658771 | 138659270 | - |
| chr3 | 138662065 | 138662564 | FOXL2NB |
| chr3 | 138662980 | 138663479 | FOXL2, FOXL2NB |
| chr3 | 142837745 | 142838244 | CHST2 |
| chr3 | 147098318 | 147098817 | - |
| chr3 | 147098335 | 147098834 | - |
| chr3 | 147105760 | 147106259 | ZIC4 |
| chr3 | 147136654 | 147137153 | - |
| chr3 | 169529777 | 169530276 | LRRC34 |
| chr3 | 169529787 | 169530286 | LRRC34 |
| chr3 | 179168510 | 179169009 | GNB4 |
| chr3 | 179168548 | 179169047 | GNB4 |
| chr3 | 183145282 | 183145781 | MCF2L2 |
| chr3 | 186490406 | 186490905 | - |
| chr4 | 785994 | 786493 | CPLX1 |
| chr4 | 41868836 | 41869335 | - |
| chr4 | 41880497 | 41880996 | - |
| chr4 | 41882330 | 41882829 | - |
| chr4 | 76555297 | 76555796 | CDKL2 |
| chr4 | 76555384 | 76555883 | CDKL2 |
| chr4 | 76555522 | 76556021 | CDKL2 |
| chr4 | 76555527 | 76556026 | CDKL2 |
| chr4 | 76555532 | 76556031 | CDKL2 |
| chr4 | 41881913 | 41882412 | - |
| chr5 | 7849953 | 7850452 | C5orf49 |
| chr5 | 7850188 | 7850687 | C5orf49 |
| chr5 | 32713473 | 32713972 | NPR3 |
| chr5 | 40680887 | 40681386 | PTGER4 |
| chr5 | 40681643 | 40682142 | PTGER4 |
| chr5 | 42950942 | 42951441 | - |
| chr5 | 42951863 | 42952362 | - |
| chr5 | 42992524 | 42993023 | - |
| chr5 | 43017435 | 43017934 | LOC648987 |
| chr5 | 43018193 | 43018692 | LOC648987 |
| chr5 | 54516555 | 54517054 | MCIDAS |

(continued)

| chr | start | end | gene |
|------|-----------|-----------|------------------|
| chr5 | 94955506 | 94956005 | GPR150 |
| chr5 | 112073100 | 112073599 | APC |
| chr5 | 112073123 | 112073622 | APC |
| chr5 | 112073148 | 112073647 | APC |
| chr5 | 112073156 | 112073655 | APC |
| chr5 | 112073176 | 112073675 | APC |
| chr5 | 112073188 | 112073687 | APC |
| chr5 | 139047755 | 139048254 | CXXC5 |
| chr5 | 139047856 | 139048355 | CXXC5 |
| chr5 | 169064201 | 169064700 | DOCK2 |
| chr5 | 170736027 | 170736526 | TLX3 |
| chr6 | 3228983 | 3229482 | TUBB2B |
| chr6 | 5026074 | 5026573 | - |
| chr6 | 5026185 | 5026684 | - |
| chr6 | 10425398 | 10425897 | - |
| chr6 | 10425849 | 10426348 | - |
| chr6 | 26235004 | 26235503 | H1-3 |
| chr6 | 26240670 | 26241169 | H4C6 |
| chr6 | 26250494 | 26250993 | H2BC9, H3C7, H4C7 |
| chr6 | 26250669 | 26251168 | H2BC9, H3C7, H4C7 |
| chr6 | 26250686 | 26251185 | H2BC9, H3C7, H4C7 |
| chr6 | 26251649 | 26252148 | H2BC9, H3C7, H4C7 |
| chr6 | 26271466 | 26271965 | H2BC10, H3C8 |
| chr6 | 26271468 | 26271967 | H2BC10, H3C8 |
| chr6 | 26271566 | 26272065 | H2BC10, H3C8 |
| chr6 | 26271577 | 26272076 | H2BC10, H3C8 |
| chr6 | 26550760 | 26551259 | - |
| chr6 | 26614399 | 26614898 | - |
| chr6 | 27462967 | 27463466 | - |
| chr6 | 27858387 | 27858886 | H2BC17, H3C12 |
| chr6 | 28411037 | 28411536 | ZSCAN23 |
| chr6 | 42738717 | 42739216 | - |
| chr6 | 42738799 | 42739298 | - |
| chr6 | 100911437 | 100911936 | SIM1 |
| chr6 | 100912656 | 100913155 | SIM1 |
| chr6 | 100912690 | 100913189 | SIM1 |
| chr6 | 100912696 | 100913195 | SIM1 |
| chr6 | 100915517 | 100916016 | SIM1 |
| chr6 | 108488085 | 108488584 | NR2E1 |

(continued)

| chr | start | end | gene |
|------|------------|------------|---------------------------------|
| chr6 | 108490645 | 108491144 | NR2E1 |
| chr6 | 108495615 | 108496114 | NR2E1 |
| chr6 | 108495735 | 108496234 | NR2E1 |
| chr6 | 133561851 | 133562350 | EYA4 |
| chr6 | 133562216 | 133562715 | EYA4 |
| chr6 | 133562220 | 133562719 | EYA4 |
| chr6 | 133562225 | 133562724 | EYA4 |
| chr6 | 133562229 | 133562728 | EYA4 |
| chr6 | 133562235 | 133562734 | EYA4 |
| chr6 | 133562242 | 133562741 | EYA4 |
| chr6 | 133562244 | 133562743 | EYA4 |
| chr6 | 1624861 | 1625360 | GMDS |
| chr6 | 26240532 | 26241031 | H4C6 |
| chr6 | 26252015 | 26252514 | H2BC9, H3C7, H4C7 |
| chr7 | 27204478 | 27204977 | HOXA10-AS, HOXA10-HOXA9, HOXA9 |
| chr7 | 27204731 | 27205230 | HOXA10-AS, HOXA10-HOXA9, HOXA9 |
| chr7 | 27204967 | 27205466 | HOXA10-AS, HOXA10-HOXA9, HOXA9 |
| chr7 | 27213734 | 27214233 | HOXA10, HOXA10-HOXA9, MIR196B |
| chr7 | 27213793 | 27214292 | HOXA10, HOXA10-HOXA9, MIR196B |
| chr7 | 27213806 | 27214305 | HOXA10, HOXA10-HOXA9, MIR196B |
| chr7 | 27232587 | 27233086 | - |
| chr7 | 27232823 | 27233322 | - |
| chr7 | 27232891 | 27233390 | - |
| chr7 | 27245292 | 27245791 | HOTTIP |
| chr7 | 27284539 | 27285038 | EVX1 |
| chr7 | 27291096 | 27291595 | - |
| chr7 | 28996389 | 28996888 | TRIL |
| chr7 | 28996652 | 28997151 | TRIL |
| chr7 | 28996923 | 28997422 | TRIL |
| chr7 | 28997235 | 28997734 | TRIL |
| chr7 | 28997616 | 28998115 | TRIL |
| chr7 | 28997828 | 28998327 | TRIL |
| chr7 | 76828635 | 76829134 | CCDC146, FGL2 |
| chr7 | 96636366 | 96636865 | DLX6, DLX6-AS1 |
| chr7 | 96636496 | 96636995 | DLX6, DLX6-AS1 |
| chr7 | 96651031 | 96651530 | DLX5 |
| chr7 | 96651865 | 96652364 | DLX5 |
| chr7 | 96651873 | 96652372 | DLX5 |
| chr7 | 117119174 | 117119673 | CFTR |

(continued)

| chr | start | end | gene |
|---|---|---|---|
| chr7 | 117119351 | 117119850 | CFTR |
| chr7 | 117119361 | 117119860 | CFTR |
| chr7 | 117119387 | 117119886 | CFTR |
| chr7 | 117119688 | 117120187 | CFTR |
| chr7 | 134143656 | 134144155 | AKR1B1 |
| chr7 | 134143669 | 134144168 | AKR1B1 |
| chr7 | 134143786 | 134144285 | AKR1B1 |
| chr7 | 143042491 | 143042990 | CLCN1 |
| chr7 | 143042548 | 143043047 | CLCN1 |
| chr7 | 151329758 | 151330257 | PRKAG2 |
| chr7 | 27252291 | 27252790 | - |
| chr7 | 27225523 | 27225524 | HOXA11 / HOXA11-AS |
| chr8 | 41424092 | 41424591 | - |
| chr8 | 49292435 | 49292934 | - |
| chr8 | 53851934 | 53852433 | NPBWR1 |
| chr8 | 57069657 | 57070156 | - |
| chr8 | 59058004 | 59058503 | FAM110B |
| chr8 | 67873093 | 67873592 | TCF24 |
| chr8 | 67873226 | 67873725 | TCF24 |
| chr8 | 67873549 | 67874048 | TCF24 |
| chr8 | 67873928 | 67874427 | TCF24 |
| chr8 | 67874116 | 67874615 | TCF24 |
| chr8 | 67874783 | 67875282 | TCF24 |
| chr8 | 70981789 | 70982288 | PRDM14 |
| chr8 | 86350318 | 86350817 | CA13, CA3 |
| chr8 | 86350331 | 86350830 | CA13, CA3 |
| chr8 | 98289898 | 98290397 | TSPYL5 |
| chr8 | 99951797 | 99952296 | OSR2, STK3 |
| chr8 | 99959473 | 99959972 | OSR2, STK3 |
| chr8 | 99959898 | 99960397 | OSR2 |
| chr8 | 99961295 | 99961794 | OSR2 |
| chr8 | 99961624 | 99962123 | OSR2 |
| chr8 | 102504197 | 102504696 | GRHL2 |
| chr8 | 102504251 | 102504750 | GRHL2 |
| chr8 | 102504314 | 102504813 | GRHL2 |
| chr8 | 102505306 | 102505805 | GRHL2 |
| chr8 | 104512833 | 104513332 | RIMS2 |
| chr8 | 11540157 | 11540656 | GATA4 |
| chr9 | 110228019 | 110228518 | - |

## Example 7. Evaluation of Marker Panel Performance in Clinical Samples

The performance of each of the marker sets (Tables 6 and 9) was evaluated by performing targeted EM-Seq on samples from 2 liver cirrhosis patients, 40 liver cancer patients, and 40 normal individuals shown in Table 14 below.

[Table 11]

|  | Normal | Liver cirrhosis | Liver cancer | Total |
|---|---|---|---|---|
| **Training** | 23 | - | 23 | 46 |
| **Validation** | 9 | - | 8 | 17 |
| **Test** | 8 | 2 | 9 | 19 |
| **Total** | 40 | 2 | 40 | 82 |

### 7-1. Targeted EM-Seq

Blood was collected from the above patients, and then subjected to first centrifugation under the conditions of 3,000 rpm, 25°C, and 10 minutes to isolate the plasma portion. Then, the plasma by isolated by the first centrifugation was subjected to second centrifugation under the conditions of 16,000g, 25°C, and 10 minutes to isolate the plasma supernatant excluding the precipitate. Cell-free DNA was extracted from the separated plasma using the Mag-bind cfDNA kit, and the concentration was measured using the Qubit DS DNA HS assay kit (Thermo Fisher Scientific, USA). For the maximum amount of the extracted cfDNA, methylation conversion was performed by replacing unmethylated cytosines with uracil using ten-eleven translocation dioxygenase 2 (TET2) and APOBEC, and then a library was prepared using enzymatic methyl-seq (NEB Kit).

The concentration and size of the prepared DNA library were measured using the Qubit DS DNA HS assay kit (Thermo Fisher Scientific, USA) and Tapestation 4200 (Agilent, USA), respectively. 200 ng of the library was pooled into groups of 8 samples, hybridization was performed, and the concentration of the captured sample was measured using the high sensitivity D1000 screen tape and reagent (Agilent, USA) with Tapestation 4200 (Agilent, USA). Sequencing was performed at a final concentration of 10 pM using the Miseq Dx (Illumina) instrument in 150 paired-end mode, generating 650X depth per sample.

### 7-2. Evaluation of Performance

Since methylated cell-free nucleic acids were sequenced, the obtained nucleic acid fragment data are in a methylated state, and can be aligned to the human reference genome, thereby identifying methylated regions in the entire human genome. In EM-Seq data, methylated cytosine remains as cytosine, and unmethylated cytosines is converted to thymines, so that methylated regions and the degree of methylation can be identified.

First, adapter trimming and quality trimming of fastq files were performed using Trim Galore (version 0.6.6), and then nucleic acid fragment data were aligned to the human reference genome (hgl9) using the Bismark (version 0.23.0) alignment tool. Only nucleic acid fragments with a mapping quality of 10 or higher were selected using the Samtools view (version 1.11) tool, and chrl-22, X, Y were retained. Then, methylation calling was performed using bismark_methylation_extractor of Bismark (version 0.23.0).

The beta values (methylation percentage) of the tumor and normal samples were merged into a single file using the methylKit (version 1.12.0) R package.

Based on this file, a CNN model for liver cancer diagnosis based on 354 markers was constructed in the same manner as in Example 5-2, and then the cancer diagnostic performance thereof was evaluated using the DPI value.

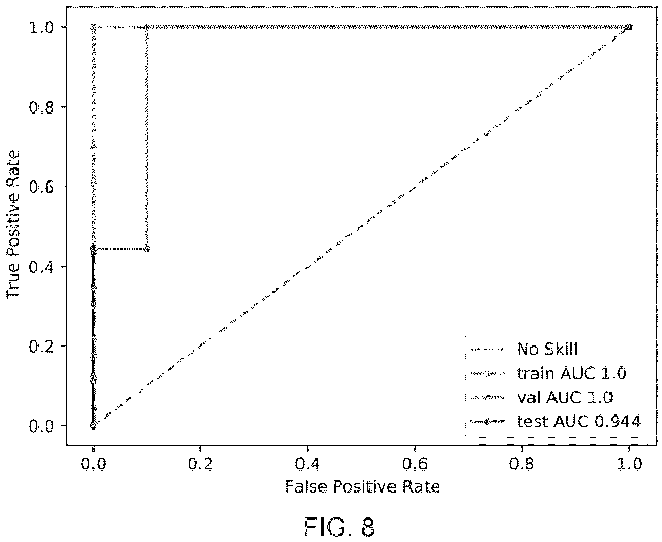

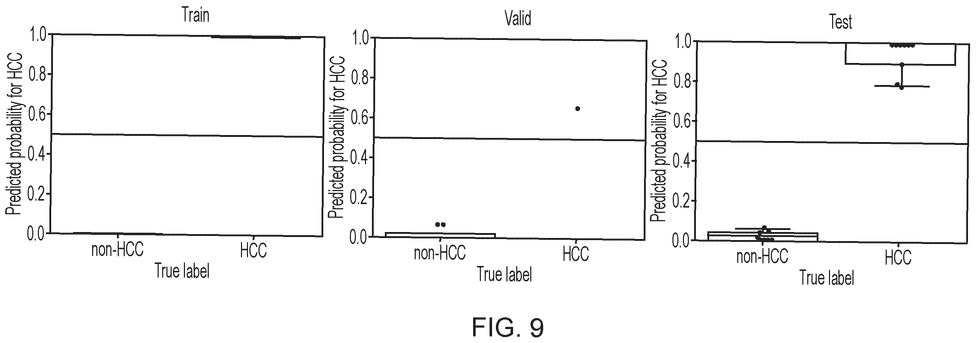

As a result, as shown in Table 12 below and FIGS. 8 and 9, it was confirmed that the accuracies were 1.00, 1.00, and 0.95 in the training, validation, and test groups, respectively, and that the AUC values, which are the results of ROC analysis, were 1.00, 1.00, and 0.94 in the training, validation, and test groups, respectively.

[Table 12]

|  | Accuracy | AUC |
|---|---|---|
| **Training** | 1.00 | 1.00 |
| **Validation** | 1.00 | 1.00 |
| **Test** | 0.95 | 0.94 |

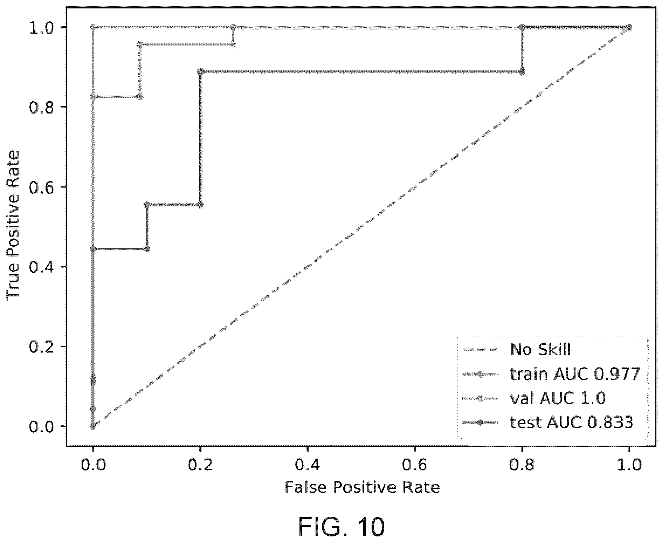

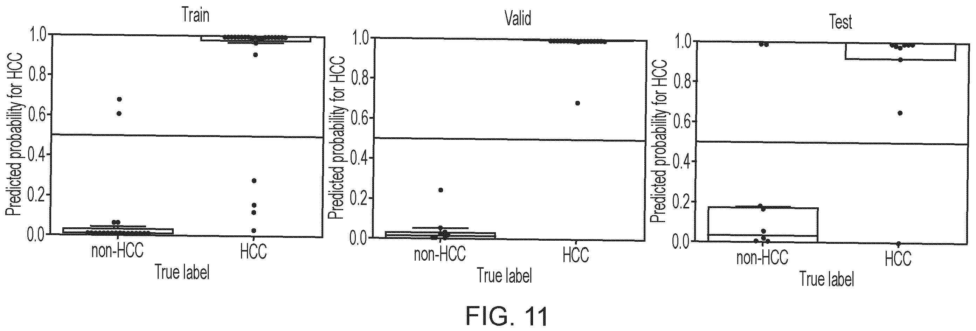

The cancer diagnostic performance of the CNN model constructed based on the EM-Seq results obtained using 14 markers in the same manner was evaluated using the DPI value. As a result, as shown in Table 13 below and FIGS. 10 and 11, it was confirmed that the accuracies were 0.87, 1.00, and 0.84 in the training, validation, and test groups, respectively, and that the AUC values, which are the results of ROC analysis, were 0.98, 1.00, and 0.83 in the training, validation, and test groups, respectively.

[Table 13]

|  | Accuracy | AUC |
|---|---|---|
| **Training** | 0.87 | 0.98 |
| **Validation** | 1.00 | 1.00 |
| **Test** | 0.84 | 0.83 |

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

**Industrial Applicability**

The DNA methylation markers for diagnosing liver cancer according to the present invention are capable of diagnosing liver cancer with high accuracy using only DNA methylation information of a blood sample without using a liver cancer tissue sample, and thus may be useful for early diagnosis of liver cancer.

**Claims**

1. A combination of DNA methylation markers for diagnosing liver cancer, comprising DNA methylation markers shown in Table 1 below:

[Table 1]

| Chromosome | Start position | End position |
|---|---|---|
| chr1 | 119532189 | 119532190 |
| chr1 | 119532195 | 119532196 |
| chr1 | 146551744 | 146551745 |
| chr2 | 45160445 | 45160446 |
| chr4 | 41882163 | 41882164 |
| chr6 | 1624978 | 1624979 |
| chr6 | 26240579 | 26240580 |
| chr6 | 26252265 | 26252266 |
| chr7 | 27252541 | 27252542 |
| chr8 | 11540407 | 11540408 |
| chr14 | 54423433 | 54423434 |
| chr14 | 100632950 | 100632951 |
| chr17 | 80291775 | 80291776 |
| chr19 | 41317067 | 41317068 |

2. The combination of DNA methylation markers for diagnosing liver cancer according to claim 1, wherein the combination of DNA methylation markers for diagnosing liver cancer further comprises DNA markers shown in Table 2 below:

[Table 2]

| Chromosome | Start position | End position |
|---|---|---|
| chr1 | 59042275 | 59042276 |
| chr2 | 208989248 | 208989249 |
| chr7 | 27225523 | 27225524 |
| chr10 | 77168431 | 77168432 |
| chr15 | 58357204 | 58357205 |
| chr18 | 32847566 | 32847567 |

3. The combination of DNA methylation markers for diagnosing liver cancer according to claim 1, wherein the combination of DNA methylation markers for diagnosing liver cancer further comprises two or more DNA methylation markers selected from the group consisting of DNA markers shown in Table 3 below:

[Table 3]

| Chromosome | Start position | End position | Chromosome | Start position | End position |
|---|---|---|---|---|---|
| chr1 | 46632446 | 46632945 | chr2 | 119067503 | 119068002 |
| chr1 | 46632621 | 46633120 | chr2 | 157177686 | 157178185 |
| chr1 | 47697715 | 47698214 | chr2 | 157178481 | 157178980 |
| chr1 | 47908984 | 47909483 | chr2 | 157178639 | 157179138 |
| chr1 | 47909931 | 47910430 | chr2 | 160761163 | 160761662 |
| chr1 | 47910206 | 47910705 | chr2 | 176987215 | 176987714 |
| chr1 | 47910593 | 47911092 | chr2 | 177003485 | 177003984 |
| chr1 | 48058627 | 48059126 | chr2 | 177003497 | 177003996 |
| chr1 | 48058711 | 48059210 | chr2 | 200331667 | 200332166 |
| chr1 | 67772878 | 67773377 | chr2 | 200331727 | 200332226 |
| chr1 | 87617460 | 87617959 | chr2 | 200331775 | 200332274 |
| chr1 | 91192216 | 91192715 | chr2 | 200333751 | 200334250 |
| chr1 | 110610649 | 110611148 | chr2 | 200334851 | 200335350 |
| chr1 | 119522605 | 119523104 | chr2 | 238583254 | 238583753 |
| chr1 | 119526861 | 119527360 | chr20 | 1784026 | 1784525 |
| chr1 | 119526906 | 119527405 | chr20 | 30778049 | 30778548 |
| chr1 | 119527634 | 119528133 | chr20 | 37433979 | 37434478 |
| chr1 | 119529680 | 119530179 | chr20 | 50720658 | 50721157 |
| chr1 | 119532523 | 119533022 | chr20 | 50721063 | 50721562 |
| chr1 | 119532675 | 119533174 | chr3 | 38080675 | 38081174 |
| chr1 | 119542807 | 119543306 | chr3 | 101497626 | 101498125 |
| chr1 | 119542966 | 119543465 | chr3 | 101497730 | 101498229 |
| chr1 | 119543086 | 119543585 | chr3 | 101497732 | 101498231 |
| chr1 | 119548277 | 119548776 | chr3 | 138658771 | 138659270 |
| chr1 | 119548575 | 119549074 | chr3 | 138662065 | 138662564 |
| chr1 | 119548602 | 119549101 | chr3 | 138662980 | 138663479 |
| chr1 | 119548895 | 119549394 | chr3 | 142837745 | 142838244 |
| chr1 | 119549013 | 119549512 | chr3 | 147098318 | 147098817 |

(continued)

| Chromosome | Start position | End position | Chromosome | Start position | End position |
|---|---|---|---|---|---|
| chr1 | 151812171 | 151812670 | chr3 | 147098335 | 147098834 |
| chr1 | 151812185 | 151812684 | chr3 | 147105760 | 147106259 |
| chr1 | 151812274 | 151812773 | chr3 | 147136654 | 147137153 |
| chr1 | 151812460 | 151812959 | chr3 | 169529777 | 169530276 |
| chr1 | 154474923 | 154475422 | chr3 | 169529787 | 169530286 |
| chr1 | 156130576 | 156131075 | chr3 | 179168510 | 179169009 |
| chr1 | 156389874 | 156390373 | chr3 | 179168548 | 179169047 |
| chr1 | 160951657 | 160952156 | chr3 | 183145282 | 183145781 |
| chr1 | 161275311 | 161275810 | chr3 | 186490406 | 186490905 |
| chr1 | 169396462 | 169396961 | chr4 | 785994 | 786493 |
| chr1 | 170629820 | 170630319 | chr4 | 41868836 | 41869335 |
| chr1 | 170630308 | 170630807 | chr4 | 41880497 | 41880996 |
| chr1 | 171810218 | 171810717 | chr4 | 41882330 | 41882829 |
| chr1 | 171810722 | 171811221 | chr4 | 76555297 | 76555796 |
| chr1 | 171811049 | 171811548 | chr4 | 76555384 | 76555883 |
| chr1 | 197882219 | 197882718 | chr4 | 76555522 | 76556021 |
| chr1 | 203598323 | 203598822 | chr4 | 76555527 | 76556026 |
| chr1 | 213123425 | 213123924 | chr4 | 76555532 | 76556031 |
| chr1 | 213123636 | 213124135 | chr5 | 7849953 | 7850452 |
| chr1 | 213123715 | 213124214 | chr5 | 7850188 | 7850687 |
| chr1 | 221064450 | 221064949 | chr5 | 32713473 | 32713972 |
| chr1 | 228645797 | 228646296 | chr5 | 40680887 | 40681386 |
| chr1 | 247171153 | 247171652 | chr5 | 40681643 | 40682142 |
| chr10 | 8094284 | 8094783 | chr5 | 42950942 | 42951441 |
| chr10 | 16562220 | 16562719 | chr5 | 42951863 | 42952362 |
| chr10 | 17271679 | 17272178 | chr5 | 42992524 | 42993023 |
| chr10 | 17271694 | 17272193 | chr5 | 43017435 | 43017934 |
| chr10 | 17271744 | 17272243 | chr5 | 43018193 | 43018692 |
| chr10 | 17271867 | 17272366 | chr5 | 54516555 | 54517054 |
| chr10 | 22541774 | 22542273 | chr5 | 94955506 | 94956005 |
| chr10 | 22625215 | 22625714 | chr5 | 112073100 | 112073599 |
| chr10 | 22765590 | 22766089 | chr5 | 112073123 | 112073622 |
| chr10 | 43697758 | 43698257 | chr5 | 112073148 | 112073647 |
| chr10 | 93647050 | 93647549 | chr5 | 112073156 | 112073655 |
| chr10 | 94834332 | 94834831 | chr5 | 112073176 | 112073675 |
| chr10 | 94834513 | 94835012 | chr5 | 112073188 | 112073687 |
| chr10 | 102894793 | 102895292 | chr5 | 139047755 | 139048254 |
| chr10 | 104000581 | 104001080 | chr5 | 139047856 | 139048355 |
| chr11 | 13689872 | 13690371 | chr5 | 169064201 | 169064700 |

EP 4 613 880 A1

(continued)

| Chromosome | Start position | End position | Chromosome | Start position | End position |
|---|---|---|---|---|---|
| chr11 | 13689910 | 13690409 | chr5 | 170736027 | 170736526 |
| chr11 | 13690452 | 13690951 | chr6 | 3228983 | 3229482 |
| chr11 | 31826324 | 31826823 | chr6 | 5026074 | 5026573 |
| chr11 | 69517049 | 69517548 | chr6 | 5026185 | 5026684 |
| chr11 | 69517221 | 69517720 | chr6 | 10425398 | 10425897 |
| chr11 | 69517591 | 69518090 | chr6 | 10425849 | 10426348 |
| chr11 | 69517753 | 69518252 | chr6 | 26235004 | 26235503 |
| chr11 | 69517947 | 69518446 | chr6 | 26240670 | 26241169 |
| chr12 | 21810029 | 21810528 | chr6 | 26250494 | 26250993 |
| chr12 | 21810380 | 21810879 | chr6 | 26250669 | 26251168 |
| chr12 | 21810558 | 21811057 | chr6 | 26250686 | 26251185 |
| chr12 | 48206598 | 48207097 | chr6 | 26251649 | 26252148 |
| chr12 | 58021319 | 58021818 | chr6 | 26271466 | 26271965 |
| chr12 | 58021463 | 58021962 | chr6 | 26271468 | 26271967 |
| chr12 | 81102236 | 81102735 | chr6 | 26271566 | 26272065 |
| chr12 | 95941619 | 95942118 | chr6 | 26271577 | 26272076 |
| chr12 | 95941738 | 95942237 | chr6 | 26550760 | 26551259 |
| chr12 | 95942511 | 95943010 | chr6 | 26614399 | 26614898 |
| chr12 | 95942657 | 95943156 | chr6 | 27462967 | 27463466 |
| chr12 | 95942714 | 95943213 | chr6 | 27858387 | 27858886 |
| chr12 | 115102476 | 115102975 | chr6 | 28411037 | 28411536 |
| chr13 | 45149778 | 45150277 | chr6 | 42738717 | 42739216 |
| chr13 | 45150012 | 45150511 | chr6 | 42738799 | 42739298 |
| chr13 | 100627091 | 100627590 | chr6 | 100911437 | 100911936 |
| chr13 | 100641159 | 100641658 | chr6 | 100912656 | 100913155 |
| chr13 | 100641396 | 100641895 | chr6 | 100912690 | 100913189 |
| chr13 | 107186620 | 107187119 | chr6 | 100912696 | 100913195 |
| chr13 | 107186927 | 107187426 | chr6 | 100915517 | 100916016 |
| chr14 | 51027611 | 51028110 | chr6 | 108488085 | 108488584 |
| chr14 | 54422525 | 54423024 | chr6 | 108490645 | 108491144 |
| chr14 | 61108957 | 61109456 | chr6 | 108495615 | 108496114 |
| chr15 | 33009281 | 33009780 | chr6 | 108495735 | 108496234 |
| chr15 | 41805280 | 41805779 | chr6 | 133561851 | 133562350 |
| chr15 | 55880644 | 55881143 | chr6 | 133562216 | 133562715 |
| chr15 | 65186056 | 65186555 | chr6 | 133562220 | 133562719 |
| chr15 | 68260324 | 68260823 | chr6 | 133562225 | 133562724 |
| chr15 | 69087559 | 69088058 | chr6 | 133562229 | 133562728 |
| chr15 | 99193679 | 99194178 | chr6 | 133562235 | 133562734 |
| chr15 | 99193743 | 99194242 | chr6 | 133562242 | 133562741 |

38

(continued)

| Chromosome | Start position | End position | Chromosome | Start position | End position |
|---|---|---|---|---|---|
| chr15 | 101459033 | 101459532 | chr6 | 133562244 | 133562743 |
| chr16 | 21170817 | 21171316 | chr7 | 27204478 | 27204977 |
| chr17 | 4981360 | 4981859 | chr7 | 27204731 | 27205230 |
| chr17 | 4981573 | 4982072 | chr7 | 27204967 | 27205466 |
| chr17 | 29297898 | 29298397 | chr7 | 27213734 | 27214233 |
| chr17 | 29297934 | 29298433 | chr7 | 27213793 | 27214292 |
| chr17 | 29298102 | 29298601 | chr7 | 27213806 | 27214305 |
| chr17 | 42030229 | 42030728 | chr7 | 27232587 | 27233086 |
| chr17 | 43338973 | 43339472 | chr7 | 27232823 | 27233322 |
| chr17 | 43339078 | 43339577 | chr7 | 27232891 | 27233390 |
| chr17 | 43339247 | 43339746 | chr7 | 27245292 | 27245791 |
| chr17 | 43339262 | 43339761 | chr7 | 27284539 | 27285038 |
| chr17 | 46655579 | 46656078 | chr7 | 27291096 | 27291595 |
| chr17 | 48636396 | 48636895 | chr7 | 28996389 | 28996888 |
| chr17 | 59529066 | 59529565 | chr7 | 28996652 | 28997151 |
| chr17 | 59529236 | 59529735 | chr7 | 28996923 | 28997422 |
| chr17 | 59534597 | 59535096 | chr7 | 28997235 | 28997734 |
| chr17 | 59534748 | 59535247 | chr7 | 28997616 | 28998115 |
| chr17 | 62777398 | 62777897 | chr7 | 28997828 | 28998327 |
| chr17 | 79480858 | 79481357 | chr7 | 76828635 | 76829134 |
| chr18 | 32847001 | 32847500 | chr7 | 96636366 | 96636865 |
| chr18 | 55019849 | 55020348 | chr7 | 96636496 | 96636995 |
| chr19 | 12305604 | 12306103 | chr7 | 96651031 | 96651530 |
| chr19 | 12305619 | 12306118 | chr7 | 96651865 | 96652364 |
| chr19 | 12305886 | 12306385 | chr7 | 96651873 | 96652372 |
| chr19 | 12305948 | 12306447 | chr7 | 117119174 | 117119673 |
| chr19 | 13209731 | 13210230 | chr7 | 117119351 | 117119850 |
| chr19 | 36736022 | 36736521 | chr7 | 117119361 | 117119860 |
| chr19 | 38182805 | 38183304 | chr7 | 117119387 | 117119886 |
| chr19 | 38754889 | 38755388 | chr7 | 117119688 | 117120187 |
| chr19 | 42901057 | 42901556 | chr7 | 134143656 | 134144155 |
| chr19 | 50553817 | 50554316 | chr7 | 134143669 | 134144168 |
| chr19 | 50554030 | 50554529 | chr7 | 134143786 | 134144285 |
| chr19 | 50554201 | 50554700 | chr7 | 143042491 | 143042990 |
| chr19 | 52207103 | 52207602 | chr7 | 143042548 | 143043047 |
| chr19 | 57018819 | 57019318 | chr7 | 151329758 | 151330257 |
| chr19 | 58220045 | 58220544 | chr8 | 41424092 | 41424591 |
| chr19 | 58220120 | 58220619 | chr8 | 49292435 | 49292934 |
| chr2 | 20068452 | 20068951 | chr8 | 53851934 | 53852433 |

(continued)

| Chromosome | Start position | End position | Chromosome | Start position | End position |
|---|---|---|---|---|---|
| chr2 | 25438860 | 25439359 | chr8 | 57069657 | 57070156 |
| chr2 | 45159843 | 45160342 | chr8 | 59058004 | 59058503 |
| chr2 | 45170072 | 45170571 | chr8 | 67873093 | 67873592 |
| chr2 | 45231217 | 45231716 | chr8 | 67873226 | 67873725 |
| chr2 | 45231532 | 45232031 | chr8 | 67873549 | 67874048 |
| chr2 | 45231641 | 45232140 | chr8 | 67873928 | 67874427 |
| chr2 | 45232167 | 45232666 | chr8 | 67874116 | 67874615 |
| chr2 | 63280819 | 63281318 | chr8 | 67874783 | 67875282 |
| chr2 | 63280889 | 63281388 | chr8 | 70981789 | 70982288 |
| chr2 | 63281067 | 63281566 | chr8 | 86350318 | 86350817 |
| chr2 | 63281133 | 63281632 | chr8 | 86350331 | 86350830 |
| chr2 | 63281594 | 63282093 | chr8 | 98289898 | 98290397 |
| chr2 | 63282452 | 63282951 | chr8 | 99951797 | 99952296 |
| chr2 | 63282763 | 63283262 | chr8 | 99959473 | 99959972 |
| chr2 | 63283717 | 63284216 | chr8 | 99959898 | 99960397 |
| chr2 | 63283816 | 63284315 | chr8 | 99961295 | 99961794 |
| chr2 | 63284518 | 63285017 | chr8 | 99961624 | 99962123 |
| chr2 | 63285799 | 63286298 | chr8 | 102504197 | 102504696 |
| chr2 | 74425262 | 74425761 | chr8 | 102504251 | 102504750 |
| chr2 | 74425330 | 74425829 | chr8 | 102504314 | 102504813 |
| chr2 | 74781846 | 74782345 | chr8 | 102505306 | 102505805 |
| chr2 | 105470311 | 105470810 | chr8 | 104512833 | 104513332 |
| chr2 | 119067387 | 119067886 | chr9 | 110228019 | 110228518 |

4. A method for providing information for diagnosing liver cancer, comprising steps of:

(a) isolating DNA from a biological sample;
(b) detecting a methylation level of the combination of DNA methylation markers according to claim 1; and
(c) determining that liver cancer is present if the detected DNA methylation marker level exceeds a cut-off value.

5. A method for diagnosing liver cancer, comprising steps of:

(a) isolating DNA from a biological sample;
(b) detecting a methylation level of the combination of DNA methylation markers according to claim 1; and
(c) determining that liver cancer is present if the detected DNA methylation marker level exceeds a cut-off value.

6. The method according to claim 4 or 5, wherein step (c) is performed by a step of determining the presence or absence of liver cancer by comparing an output result value, obtained by inputting and analyzing information on the detected methylation level of the combination of DNA methylation markers into an artificial intelligence model trained to diagnose liver cancer, with a cut-off value.

7. The method according to claim 4 or 5, wherein detecting the methylation level in step (b) is performed using any one method selected from the group consisting of PCR, methylation-specific PCR, real-time methylation-specific PCR, PCR using methylated DNA-specific binding protein, quantitative PCR, PCR using methylation-specific PNA, melting curve analysis, DNA chip assay, pyrosequencing, bisulfite sequencing, and methylation next-generation sequencing.

8. A composition for diagnosing liver cancer, comprising a combination of primers capable of amplifying each DNA methylation marker of the combination of DNA methylation markers according to any one of claims 1 to 3.

9. A composition for diagnosing liver cancer, comprising a combination of probes, each capable of specifically hybridizing to either a polynucleotide comprising at least 10 contiguous nucleotides, which contains a methylated base of a DNA methylation marker of the combination of DNA methylation markers according to any one of claims 1 to 3, or a polynucleotide complementary thereto.

10. A kit for diagnosing liver cancer, comprising the composition according to claim 8 or 9.

FIG. 1

FIG. 2

**FIG. 3**

N marker

| CpG1 | CpG20 | CpG55 | . | . | . | . | CpG655 |

| CpG1 | CpG89 | CpG104 | . | . | . | . | CpG701 |

.
.
.

| CpG1 | CpG13 | CpG52 | . | . | . | . | CpG356 |

1,000 random sets

⟱ Σ Beta value

|     | Set 1 | Set 2 | Set 3 | . | . | . | . | Set 1000 |
|-----|-------|-------|-------|---|---|---|---|----------|
| N1  | 1.212 | 2.371 | 4.194 | . | . | . | . | 1.002    |
| N2  | 2.129 | 2.621 | 3.298 | . | . | . | . | 2.401    |
| N3  | 1.009 | 1.093 | 1.826 | . | . | . | . | 1.552    |
| C1  | 5.123 | 4.945 | 3.448 | . | . | . | . | 2.676    |
| C2  | 1.826 | 2.552 | 6.833 | . | . | . | . | 9.234    |
| C3  | 3.448 | 7.981 | 1.093 | . | . | . | . | 7.245    |

⟱ AUC

|     | CpG1 | CpG20 | CpG55 | . | . | . | . | CpG655 |
|-----|------|-------|-------|---|---|---|---|--------|
| AUC | 0.88 | 0.97  | 0.23  | . | . | . | . | 0.02   |

FIG. 4

FIG. 5

FIG. 6

FIG. 7

FIG. 8

FIG. 9

FIG. 10

FIG. 11

## INTERNATIONAL SEARCH REPORT

| International application No. |
| --- |
| **PCT/KR2023/017175** |

**A. CLASSIFICATION OF SUBJECT MATTER**

**C12Q 1/6886**(2018.01)i; **G16B 40/00**(2019.01)i

According to International Patent Classification (IPC) or to both national classification and IPC

**B. FIELDS SEARCHED**

Minimum documentation searched (classification system followed by classification symbols)

C12Q 1/6886(2018.01); C12Q 1/68(2006.01); C12Q 1/6813(2018.01); C12Q 1/6876(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 간암(liver cancer), 메틸화(methylation), 마커(marker), 진단(diagnosis), 염색체 (chromosome), 프라이머(primer), TBX15, LINC00682, GMDS, H4C6

**C. DOCUMENTS CONSIDERED TO BE RELEVANT**

| Category* | Citation of document, with indication, where appropriate, of the relevant passages | Relevant to claim No. |
| --- | --- | --- |
| A | KR 10-2022-0069869 A (INDUSTRY-ACADEMIC COOPERATION FOUNDATION, YONSEI UNIVERSITY et al.) 27 May 2022 (2022-05-27)<br>See claim 1. | 1-9 |
| A | KR 10-2021-0044441 A (GENCURIX INC.) 23 April 2021 (2021-04-23)<br>See claims 1-9. | 1-9 |
| A | WO 2022-204358 A1 (FREENOME HOLDINGS, INC.) 29 September 2022 (2022-09-29)<br>See entire document. | 1-9 |
| A | EP 3950960 A1 (LEPIDYNE CO., LTD.) 09 February 2022 (2022-02-09)<br>See entire document. | 1-9 |
| A | JP 2022-501033 A (BLUESTAR GENOMICS, INC.) 06 January 2022 (2022-01-06)<br>See entire document. | 1-9 |

☐ Further documents are listed in the continuation of Box C.  ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **05 February 2024** | **05 February 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/017175** |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
      because they relate to subject matter not required to be searched by this Authority, namely:

2. ☐ Claims Nos.:
      because they relate to parts of the international application that do not comply with the prescribed requirements to such an extent that no meaningful international search can be carried out, specifically:

3. ☑ Claims Nos.: **10**
      because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/KR2023/017175**

| Patent document cited in search report | | | Publication date (day/month/year) | Patent family member(s) | | | Publication date (day/month/year) |
|---|---|---|---|---|---|---|---|
| KR | 10-2022-0069869 | A | 27 May 2022 | None | | | |
| KR | 10-2021-0044441 | A | 23 April 2021 | None | | | |
| WO | 2022-204358 | A1 | 29 September 2022 | KR | 10-2023-0162662 | A | 28 November 2023 |
| | | | | US | 2023-0178181 | A1 | 08 June 2023 |
| EP | 3950960 | A1 | 09 February 2022 | CN | 113544288 | A | 22 October 2021 |
| | | | | EP | 3950960 | A4 | 18 January 2023 |
| | | | | JP | 2022-522354 | A | 18 April 2022 |
| | | | | JP | 7340879 | B2 | 08 September 2023 |
| | | | | KR | 10-2068310 | B1 | 20 January 2020 |
| | | | | US | 2023-0109129 | A1 | 06 April 2023 |
| | | | | WO | 2020-175903 | A1 | 03 September 2020 |
| JP | 2022-501033 | A | 06 January 2022 | CN | 113508181 | A | 15 October 2021 |
| | | | | EP | 3853383 | A1 | 28 July 2021 |
| | | | | US | 2020-0123616 | A1 | 23 April 2020 |
| | | | | WO | 2020-061380 | A1 | 26 March 2020 |
| | | | | WO | 2020-061380 | A9 | 28 May 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1557183

- KR 1191947

**Non-patent literature cited in the description**

- **CROSS S. et al.** *Curr. Opin. Gene Develop.*, 1995, vol. 5, 309

- **METZKER, M.** *Nature Biotechnology Reviews*, 2010, vol. 11, 31-46